(19) Europäisches Patentamt
European Patent Office
Office européen des brevets

(11) EP 0 976 723 A2

(12) EUROPEAN PATENT APPLICATION

(43) Date of publication:
**02.02.2000 Bulletin 2000/05**

(51) Int Cl.[7]: **C07C 265/14**, C07C 263/04, C08G 18/75, C09J 175/04

(21) Application number: **99401948.7**

(22) Date of filing: **30.07.1999**

(84) Designated Contracting States:
**AT BE CH CY DE DK ES FI FR GB GR IE IT LI LU MC NL PT SE**
Designated Extension States:
**AL LT LV MK RO SI**

(30) Priority: **31.07.1998 JP 23008598**
**03.08.1998 JP 23121298**
**03.08.1998 JP 23121398**
**03.08.1998 JP 23121498**
**03.08.1998 JP 23121598**

(71) Applicant: **Daicel Chemical Industries, Ltd.**
**Sakai-shi, Osaka-fu (JP)**

(72) Inventors:
- **Tanaka, Yasutaka**
  **Himeji-shi, Hyogo-ken (JP)**
- **Miho, Takuya**
  **Kuga-gun, Yamaguchi-ken (JP)**
- **Yagii, Toyokazu**
  **Ashiya-shi, Hyogo-ken (JP)**

(74) Representative: **Portal, Gérard et al**
**Cabinet Beau de Loménie**
**158, rue de l'Université**
**75340 Paris Cédex 07 (FR)**

## (54) A cycloaliphatic polyisocyanate compound, a process for the preparation thereof, a polyurethane therefrom, and an adhesive composition

(57) The invention relates to a cycloaliphatic polyisocyanate represented by general formula (1) described below,

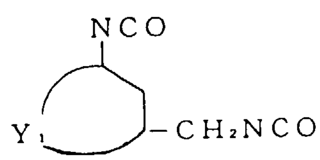

(1)

in which Y1 is a divalent saturated aliphatic hydrocarbon residual group, for example, 3-isocyanatemethyl-5,5-dimethylcyclohexylisocyanate.

The invention also relates to a process for manufacturing the above polyisocyanate by a reaction for conversion into an isocyanate of 3-aminomethylcycloalkyl amines which is obtained by a reductive amination of 3-formylcycloalkanones or 3-formylcycloalkenones obtained by oxidation of 3-methyl group in 3-methylcycloalkanones or 3-methylcycloalkenones. Further, 3-isocyanatemethyl-5,5-dimethylcyclohexylisocyanate having the chlorine content of not more than 10 ppm is optionally obtained using dimethylcarbonate by a phosgene method or a nonphosgene method.

EP 0 976 723 A2

## Description

### FIELD OF THE INVENTION

The present invention relates to a novel cycloaliphatic polyisocyanate, a process for the preparation thereof, a composition primarily containing thereof, and a polyurethane using the cycloaliphatic polyisocyanate. In more detail, the present invention relates to a novel cycloaliphatic polyisocyanate such as 3-isocyanatemethyl-5,5-dimethyl-cyclohexylisocyanate which is high in reactivity and has a unique property owing to the presence of isocyanatemethyl group which is harmless, and the cycloaliphatic polyisocyanate in which the content of chlorine components is not more than 10 ppm, a process for the preparation thereof, and a composition containing the cycloaliphatic polyisocyanate which is a primary component, and acid components such as a protonic acid and/or a Lewis acid, and moreover, a polyurethane resin having a unique property in which the cycloaliphatic polyisocyanate is employed, and an adhesive composition which comprises the polyurethane resin.

### BACKGROUND OF THE INVENTION

Polyisocyanates have been employed as coatings and polyurethane resins, etc., by allowing to react with a compound having active hydrogens such as hydroxyl group or amino group.

Particularly, isophorone diisocyanate has been widely employed in industrial fields over wide fields such as paints, a binder for an ink, coatings, and adhesives. However, since poisonous cyanic acid is employed as a raw material for the preparation, it is problematic in view of an industrial process for the preparation.

On the other hand, since a polyurethane resin has a disadvantage that it is poor in weatherability, whereby, it is apt to readily yellow. Isophorone diisocyanate which is a nonyellowing type one has been employed for uses in which weatherability is required. However, since isophorone diisocyanate has a disadvantage that a reaction rate is slow in a reaction for the conversion into a polyurethane with a polyol having hydroxyl groups, there has been desired a development of a polyisocyanate in which the disadvantage is improved. Further, all the polyisocyanates are industrially manufactured by a reaction of an amine compound with phosgene at present time.

As is well known, phosgene is a reactive compound having a high selectivity, however, it has a strong toxicity at the same time, and a severe control is required when handling this compound.

So long as the preparation using phosgene is continued, the risk of damage caused by a leakage of phosgene cannot be avoided.

Also, the polyisocyanate compound prepared by a reaction of an amine compound with phosgene inevitably contains impurities such as unreacted phosgene, chloroformate which is a by-product, a mono and dicarbamoyl chloride which are an intermediate.

Further, it contains hydrogen chloride or chlorine as impurities which is a by-product in a phosgenization reaction.

Usually, chlorine or chlorine compounds are contained in a salient amount, i.e., several hundreds ppm. Even though it contains chlorine or chlorine compounds in a polyisocyanate compound in the above-described concentration, there is a problem as described below.

That is, it is introduced into a product in a polyurethane industry which is a largest use of an isocyanate compound, and it affects a variety of harms.

For example, the monocarbamoyl chloride compound produces a terminal group in a reaction for the conversion into a polyurethane of a polyisocyanate compound with a diol, and it interferes highly-polymerizing of a polyurethane.

Further, the above-described chlorine or chlorine compounds react with water or moisture in air, and it partially changes to chlorine ion. The chlorine ion also diversely affects in the preparation or the use of a variety of polyurethanes. For example, in the case of a reaction for the conversion into a polyurethane of a polyisocyanate, there are employed catalysts for a reaction for the conversion into a polyurethane such as tin compounds which include dibutyltin dilaurate and dioctyltin dilaurate, metallic salts such as manganese acetate, lead octylate, and zinc octylate, tertiary amines such as diazabicyclo undecene, triethylamine, diazabicyclo octane, and diethyl aminoethanol which are classified in Lewis bases, and the catalysts are neutralized or decomposed in the presence together with chlorine ion.

Accordingly, in the case of employing a polyisocyanate compound prepared by a method using phosgene, the catalysts for a reaction for the conversion into a polyurethane must be added at an amount exceeding an amount consumed by chlorine ion, resulting in that an excessive amount of catalysts are employed.

Further, chlorine is contained in a urethane coating in which a polyisocyanate compound is employed, and it becomes apt to readily cause a corrosion in metallic materials coated by the urethane coating and blisters in a coated layer itself.

As described hereinabove, a polyisocyanate compound containing chlorine or chlorine compounds includes

some problems in the preparation or the uses thereof.

It is to be noted that there is not found out a method for economically removing chlorine from the cycloaliphatic polyisocyanate compound represented by general formula (1), at present time.

Many kinds of polyisocyanate compounds are manufactured by a conversion into an isocyanate of an amine using phosgene except ones having a special structure.

Of the polyisocyanate compounds, diisocyanate compounds are allowed to react with a compound having active hydrogens such as hydroxyl group or amino group to obtain a polyurethane resin, and the polyurethane resin is widely used as many products.

The following reaction mechanism [a reaction scheme (1)] is described in the Journal of Paint Technology, Vol. 43, No. 562 (1971) or ACS symposium, Ser., 270, 111 (1985) as a mechanism of the conversion from an isocyanate compound into a polyurethane resin.

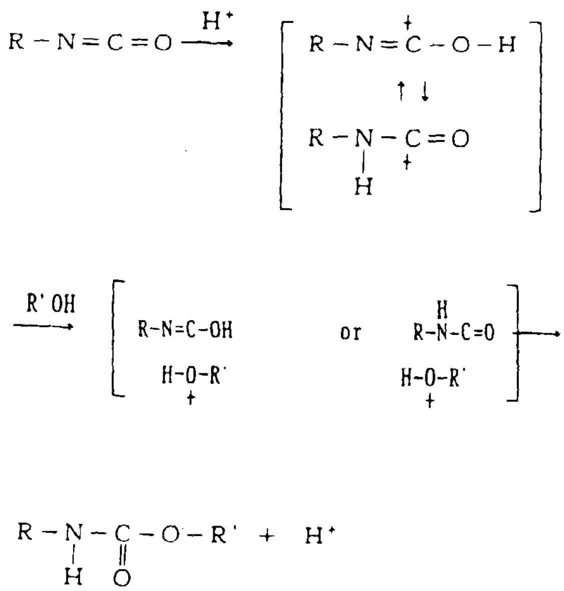


That is, it is presumed that the proton which exists in the reaction system catalyzes the electrophilic attack of the carbonyl group in the isocyanate compound on a hydroxyl group.

In order to investigate conventional polyisocyanates and a process for the preparation thereof, as described hereinabove, most of the conventional polyisocyanates are directly prepared by a reaction of an amine with phosgene, so that they contain not only a slight amount of unreacted phosgene but also compounds having a functional group such as a carbamoyl or carboxyl group and hydrogen chloride as impurities.

The content of the impurities in the conventional polyisocyanates ranges in 100-1,000 ppm in the form of chlorine.

Accordingly, it is presumed that the proton eliminated from these impurities catalyzes the conversion of an isocyanate into a urethane. However, the content of the chlorine compounds in a polyisocyanate compound prepared from a dialkyl carbonate and a diamine is estimated to be as follows. That is, the content of the chlorine compounds in a polyisocyanate compound prepared from a dialkyl carbonate prepared through phosgene and a diamine ranges in 5-50 ppm in the form of chlorine, while that in a polyisocyanate compound prepared from a dialkyl carbonate prepared without using phosgene and a diamine through a urethane compound is 1 ppm or below. Such the polyisocyanates compound containing only a slight amount of chlorine compounds is low in reactivity of the conversion into a polyurethane.

In JP-A-03275661 or JP-A-03287570 Official Gazette, there is proposed a method for acceleration of a reactivity by a protonic acid and, for example, in the case of preparing a polyurethane resin for an electronic material which exceedingly evades acid components, easygoing addition of the protonic acid is not preferred.

Polyurethanes have been widely employed for uses of adhesives as a mixture with other components or a composition allowed to react with other compounds due to excellent properties, as described below.

(a) Adhesives for a laminate film are described in JP-B-89011233, JP-B-89012318, JP-A-61047775, JP-A-61209282, and JP-A-63196678 Official Gazettes. Ink binders for printing are described in JP-B-87003188, JP-A-59147066, JP-A-61066769, JP-A-62220568, and JP-A-63165472 Official Gazettes.

(b) Curable adhesives for optical fibers, a large size Braun tube, magnetic materials, etc., which comprise an ultraviolet ray-curable or electronic beam-curable urethane(meth)acrylate which is prepared by a reaction of a polyisocyanate compound with a (meth)acrylate containing hydroxyl group are described in JP-A-60051766, JP-A-60051713, and JP-B-88066846 Official Gazettes.

(c) Polyurethane-made adhesive sheets and laminates are described inJP-A-61177241, JP-A-61179733, JP-A-61281136, JP-A-61225271, JP-A-61225272, JP-A-61225273, JP-A-61223078, and JP-A-63132922 Official Gazettes.

(d) A reactive adhesive which comprises an isocyanate prepolymer composition is described in JP-A-61136570, JP-A-61145268, and JP-B-85058269 Official Gazettes.

(e) A primer composition for a urethane resin composition in which there is employed a urethane adduct obtained by a reaction of a polyisocyanate compound obtained by a buret reaction and/or an isocyanurate reaction or an isocyanate adduct obtained by a reaction of a diol or triol having a low molecular weight are described in JP-A-61296075, JP-A-62053337, JP-A-61278582, and JP-A62233022 Official Gazettes.

(f) An adhesive composition which comprises a urethane-modified epoxy resin is described in JP-A-61228015, JP-A-01146978, JP-A-01146979, and JP-A-01146980 Official Gazettes.

(g) An adhesive composition in which a polyurethane is dispersed in an aqueous medium is described in JP-B-89020270, JP-A-60141711, JP-A-60233120, JP-A-60243163, and JP-A-61098720 Official Gazettes.

(h) An adhesive composition for plastic films, etc. is described in JP-B-89007633, JP-A-62030176, JP-A-63179987, JP-A-01108286, and JP-A-02084482 Official Gazettes.

The urethane adhesives are usually prepared by a polyaddition reaction in which there are employed a polyisocyanate compound and a polyol such as a polyetherpolyol, polyesterpolyol, and a polyvalent alcohol having a low molecular weight, or there is further optionally employed a compound having active hydrogens which is a chain extender.

Of those, a great part of the polyisocyanate compounds are industrially prepared by a direct conversion into an isocyanate of a polyamine using phosgene except a compound having a special structure. That is, it results in that at least one of starting raw materials are prepared using poisonous phosgene in any one step of preparation processes.

In the above-mentioned field of a variety of the adhesives, weatherability, corrosion resistance, and heat resistance of polyurethanes which are a primary component are important physical properties, and there has been desired an appearance of a polyurethane having more improved physical properties.

As described hereinabove, in the case that at least one of starting raw materials are prepared using poisonous phosgene in any one step of preparation processes, there is maintained a state that chlorine compounds become inevitably contained in a polyurethane product produced by the starting raw materials.

When the polyurethane containing chlorine compounds is employed, in particular, weatherability and corrosion resistance are adversely affected in the physical properties.

In a polyisocyanate compound prepared by a direct isocyanation of a polyamine with phosgene, unreacted phosgene and a variety of chlorine compounds such as chloroformate which is a by-product in the reaction or dicarbamoylchloride which is an intermediate in the reaction are mixed in the concentration of several hundreds ppm.

And, the chlorine compounds cannot be removed by an economical method. Further, also in a polyisocyanate compound prepared by a polyamine with a dialkylcarbonate prepared using poisonous phosgene, although it is a lower content than in a polyisocyanate compound directly prepared by using the above-described phosgene method, a variety of chlorine compounds are remained in the concentration of several tens ppm.

Accordingly, although there is fairly reduced an adverse affection to weatherability and corrosion resistance, it is not completely improved.

In view of the above-described situation, an aim of the present invention is to provide,

(1) A cycloaliphatic polyisocyanate which is a novel compound, and which has characteristic properties such as a high reactivity with hydroxyl group, etc.,

(2) A cycloaliphatic polyisocyanate having a lower chlorine content,

(3) A process for the preparation of a cycloaliphatic polyisocyanate which does not include problems in the preparation,

(4) A composition primarily containing the cycloaliphatic polyisocyanate having a high reactivity,

(5) A composition primarily containing the cycloaliphatic polyisocyanate having a high reactivity without containing a protonic acid,

(6) A polyurethane resin having a high weatherability such as nonyellowing, and

(7) An adhesive composition comprising a polyurethane resin having excellent weatherability, corrosion resistance,

and heat resistance.

It is to be noted that the present invention is classified as follows.

(a) A cycloaliphatic polyisocyanate and a process for the preparation of thereof.
(b) A process for the preparation of a cycloaliphatic polyisocyanate by a method through a carbamate, and the cycloaliphatic polyisocyanate having chlorine content of not more than 10 ppm.
(c) A cycloaliphatic polyisocyanate composition containing a protonic acid.
(d) A cycloaliphatic polyisocyanate composition containing a Lewis acid.
(e) A polyurethane using the above-described cycloaliphatic polyisocyanate.
(f) An adhesive composition comprising a polyurethane using the above-described cycloaliphatic polyisocyanate or a cycloaliphatic polyisocyanate composition containing an acid.

## SUMMARY OF THE INVENTION

The present inventors, as a result of an intensive investigation in order to solve the above-described problems, have found that a cycloaliphatic polyisocyanate can attain the purpose of the present invention, which is obtained by introducing a formyl group through oxidation of a methylcycloalkanone having a specified structure, and a reductive amination thereof, and then converting an amino group in a cycloaliphatic polyamine obtained into an isocyanate group, and a first aspect of the present invention has been completed.
Also, the present inventors, as a result of an intensive investigation in order to solve the above-described problems, have found that a cycloaliphatic polyisocyanate having a lower chlorine content can be obtained by preparing a carbamate compound through a reaction of the above-described diamines with a dialkylcarbonate compound prepared without using phosgene, and then by preparing a cycloaliphatic polyisocyanate through a thermal decomposition of the carbonate compound, and a second aspect of the present invention has been completed.
And also, the present inventors, as a result of an intensive investigation in order to allow to show a reactivity of a cycloaliphatic polyisocyanate having a lower chlorine content identically to or more exceeding a polyisocyanate compound having the chlorine content of 100-1,000 ppm or so prepared from phosgene and a diamine, have found that a reaction for the conversion into a polyurethane is surprisingly very activated with the use of a composition primarily containing a polyisocyanate in which a protonic acid is added which can emit proton, and a third aspect of the present invention has been completed.
And also, the present inventors, as a result of an intensive investigation in order to allow to show a reactivity of a cycloaliphatic polyisocyanate having a lower chlorine content prepared without using phosgene even in any stages identically to or more exceeding a polyisocyanate compound having the chlorine content of 100-1,000 ppm or so which is prepared from phosgene and a diamine, have found that a reaction for the conversion into a polyurethane is surprisingly very activated with the use of a composition primarily containing a polyisocyanate in which a Lewis acid is added which can emit proton, and a urethane prepared by the reaction does not contain a compound having functional groups such as carbamoyl group, carboxylic group, hydrogen chloride, and compounds derived therefrom in addition to phosgene, whereby physical properties such as weatherability and corrosion resistance, and heat resistance are improved, and a urethane which can be preferably employed as coatings, adhesives, and molded articles can be supplied, and a fourth aspect of the present invention has been completed.
Also, the present inventors, as a result of an intensive investigation, have found that a nonyellowing polyurethane can be obtained by using a cycloaliphatic polyisocyanate obtained through transforming amino groups in a cycloaliphatic polyamine into isocyanate groups. The cycloaliphatic polyamine is obtained by oxidation of methylcycloalkanone having a specified structure to introduce formyl groups, and then by a reductive amination thereof, and a fifth aspect of the present invention has been completed.
Also, the present inventors, as a result of an intensive investigation, have found that an adhesive composition is very excellent in view of weatherability, corrosion resistance, and heat resistance which comprises a polyurethane composition prepared using a specified cycloaliphatic polyisocyanate which is prepared without substantially using phosgene in any steps of the preparation processes of starting raw materials, and a sixth aspect of the present invention has been completed.
That is, the first aspect of the present invention provides a cycloaliphatic polyisocyanate represented by general formula (1) described below,

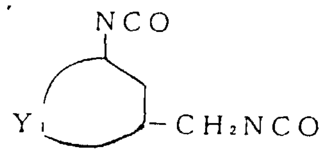

(1)

(wherein, Y1 is a divalent saturated aliphatic hydrocarbon residual group).

The second aspect of the present invention provides a cycloaliphatic polyisocyanate described in the first aspect of the present invention, wherein Y1 in the formula (1) is an alkylene group having a carbon number of 1-10.

The third aspect of the present invention provides a cycloaliphatic polyisocyanate described in the first aspect of the present invention, wherein the cycloaliphatic polyisocyanate represented by the general formula (1) is 3-isocyanatemethyl-5,5-dimethylcyclohexylisocyanate.

The fourth aspect of the present invention provides a cycloaliphatic polyisocyanate described in the three first aspects of the present invention, characterized in that the chlorine content is not more than 10 ppm.

The fifth aspect of the present invention provides a cycloaliphatic polyisocyanate described in one of the four first aspects of the present invention, characterized by further subjecting 3-aminomethyl cycloalkylamines represented by general formula (4) to a reaction for the conversion into an isocyanate after a reductive amination of 3-formyl cycloalkanones represented by general formula (2) or 3-formylcycloalkenones represented by general formula (3);

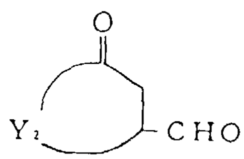

(2)

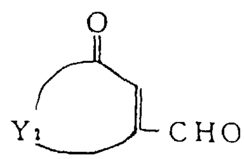

(3)

(wherein, Y2 is a divalent saturated or unsaturated aliphatic hydrocarbon residual group)

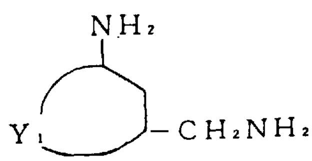

(4) ,

(wherein, Y1 is a divalent saturated aliphatic hydrocarbon residual group)

The sixth aspect of the present invention provides a process according to the fifth aspect wherein the preparation of the cycloaliphatic polyisocyanate described in one of the four first aspects of the present invention, in which the reductive amination is carried out by introducing hydrogen into a reaction system containing 3-formylcycloalkanones or 3-formylcycloalkenones, ammonia, and a solvent under the presence of a catalyst.

The seventh aspect of the present invention provides a cycloaliphatic polyisocyanate described in the fifth or sixth aspect of the present invention, wherein there are employed 3-formylcycloalkanones represented by general formula (2) or 3-formylcycloalkenones represented by general formula (3) produced by oxidation of 3-methylcycloalkanones represented by general formula (5) or 3-methylcycloalkenones represented by general formula (6);

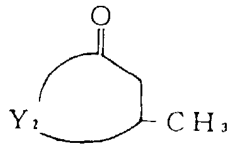
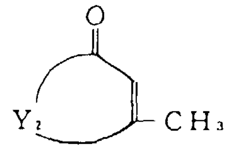 ,

(5) (6)

(wherein, Y2 is a divalent saturated or unsaturated aliphatic hydrocarbon residual group).

The eighth aspect of the present invention provides a cycloaliphatic polyisocyanate described in the fifth aspect of the present invention, characterized in that the isocyanation is carried out by thermal decomposition of a dialkylcarbamate represented by formula (7) obtained by the reaction of 3-aminomethylcycloalkyl amines with a dialkylcarbonate prepared without using phosgene through a reaction step;

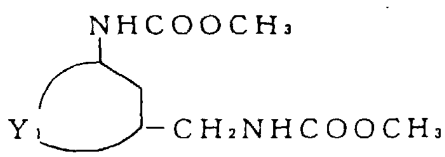

(7)

(wherein, Y1 is a divalent saturated aliphatic hydrocarbon residual group).

The ninth aspect of the present invention provides a process for the preparation of the cycloaliphatic polyisocyanate described in the eighth aspect of the present invention, wherein the alkylcarbonate is dimethylcarbonate.

The tenth aspect of the present invention provides a process for the preparation of the cycloaliphatic polyisocyanate described in the ninth aspect of the present invention, wherein the dimethylcarbonate is prepared from methanol, oxygen, and carbon monoxide.

The eleventh aspect of the present invention provides a process for the preparation of the cycloaliphatic polyisocyanate described in the ninth aspect of the present invention, wherein the dimethylcarbonate is a product in which propylene carbonate prepared by propylene oxide and carbon dioxide is further allowed to react with methanol.

The twelfth aspect of the present invention provides a composition primarily containing the cycloaliphatic polyisocyanate described in any one of the first-fourth aspects of the present invention, and containing a protonic acid and/or a Lewis acid.

The thirteenth aspect of the present invention provides a composition primarily containing a cycloaliphatic polyisocyanate described in any one of the first-fourth aspects of the present invention, and containing a Lewis acid.

The fourteenth aspect of the present invention provides a composition primarily containing a cycloaliphatic polyisocyanate described in fourth aspect of the present invention, and containing a Lewis acid.

The fifteenth aspect of the present invention provides a composition primarily containing a cycloaliphatic polyisocyanate described in twelfth or thirteenth or fourteenth aspect of the present invention, wherein the addition amount of an acid is $1x10^{-8}$ to $10^{-1}$ equivalent based on 1 mol of an isocyanate group in the cycloaliphatic polyisocyanate.

The sixteenth aspect of the present invention provides a polyurethane obtained by a reaction of cycloaliphatic polyisocyanates described in the 1-4 aspects of the present invention with polyols and/or polyamines.

The seventeenth aspect of the present invention provides a polyurethane obtained by a reaction of a cycloaliphatic polyisocyanate composition described in any one of the 13 and 14 aspects of the present invention with polyols and/or polyamines.

The eighteenth aspect of the present invention provides an adhesive composition comprising the polyurethane described in the 16 or 17 aspect of the present invention.

## DETAILED DESCRIPTION OF THE PREFERRED EMBODIMENT

Hereinafter, the present invention is illustrated in detail.

I. A cycloaliphatic polyisocyanate and a process for the preparation thereof

[(I-1) Cycloaliphatic polyisocyanate]

The cycloaliphatic polyisocyanate in the present invention is represented by the above-described general formula (1).

In the general formula (1), Y1 is a divalent saturated or unsaturated aliphatic hydrocarbon residual group which may have a linear or a branched chain, and the branched chain may be substituted by two alkyl groups at identical carbon atoms in a linear-state hydrocarbon group.

As the divalent saturated aliphatic hydrocarbon residual group, there can be enumerated, for example, an alkylene group having a carbon number of 1-10 such as methylene group, ethylene group, propylene group, trimethylene group, 2-methyltrimethylene group, 2,2-dimethyltrimethylene group, tetramethylene group, 2-methyltetramethylene group, 2,2-dimethyltetramethylene group, pentamethylene group, and hexamethylene group.

Preferred alkylene group having a linear or a branched chain is an alkylene group having a carbon number of 2-8, in particular, an alkylene group having a carbon number of 2-6. Specifically, the alkylene group is 2,2-dimethyltrimethylene group, and as the cycloaliphatic polyisocyanate represented by the general formula (1), 3-isocyanatemethyl-5,5-dimethylcyclohexylisocyanate can be enumerated.

In the aliphatic hydrocarbon residual groups, any positions may be substituted by a variety of substituents such as, for example, amino group, hydroxyl group, an alkoxy group having a carbon number of 1-4, carboxyl group, an alkoxy carbonyl group, cycloaliphatic hydrocarbon groups (a cycloalkyl group, a cycloalkenyl group, and a cycloalkinyl group, etc.), aromatic hydrocarbon groups (a aryl group such as a phenyl group), a halogen group, and nitro group, etc.

Subsequently, there is illustrated a process for the preparation of the cycloaliphatic polyisocyanate represented by the above-described general formula (1) according to the present invention.

The cycloaliphatic polyisocyanate represented by the above-described general formula (1) can be prepared by an isocyanation after a reductive amination of 3-formylcycloalkanones represented by the above-described general formula (2) or 3-formylcycloalkenones represented by the above-described general formula (3).

As the cycloaliphatic polyisocyanate which can be prepared, there are enumerated an isocyanate compound such as 3-isocyanate methylcyclopentylisocyanate, 3-isocyanatemethylcyclohexyl isocyanate, 3-isocyanatemethyl-5-methylcyclohexylisocyanate, 3-isocyanatemethylcycloheptylisocyanate, 3-isocyanatemethyl-5,5-dimethylcyclohexylisocyanate, 3-isocyanatemethylcyclooctyl isocyanate, 3-isocyanatemethyl-5-methylcyclooctylisocyanate, and 5-phenyl-3-isocyanatemethylcyclohexyl isocyanate.

[(I-2) 3-formylcycloalkanones and 3-formylcycloalkenones]

In the present invention, a compound to be employed as a substrate for the above-described reductive amination is represented by the above-described general formula (2) or (3).

The compound represented by the general formula (2) is preferably 3-formylcycloalkanones, and the compound represented by the general formula (3) is preferably 3-formylcycloalkenones. Both compounds are a cyclic ketone having formyl group at beta-position.

In the general formulae (2) and (3), Y2 is a divalent saturated or unsaturated aliphatic hydrocarbon residual group, and it may have a linear or branched chain, and the branched chain may be substituted by two alkyl groups at identical carbon atoms in a linear-state hydrocarbon group.

In the case that Y2 is a divalent saturated aliphatic hydrocarbon residual group, it is identical to the divalent saturated aliphatic hydrocarbon residual group in the above-described Y1.

In the case that Y2 is a divalent unsaturated aliphatic hydrocarbon residual group, there can be enumerated, for example, an alkylene group such as propenylene group, 2-methylpropenylene group, 2,2-dimethylpropenylene group, butylene group, and pentylene group.

Preferred linear or branched chain alkylene group or alkenylene group is an alkylene group or alkenylene group having a carbon number of 2-8, in particular, it is an alkylene group or alkenylene group having a carbon number of 2-6.

As specific examples of the 3-formylcycloalkanones represented by the general formula (2), there can be enumerated 3-formylcyclopentanone, 3-formylcyclohexanone, 5-methyl-3-formylcyclohexanone, 3-formylcycloheptanone, 5,5-dimethyl-3-formylcyclohexanone, 3-formylcyclooctanone, 5-methyl-3-formylcyclooctanone, and 5-phenyl-3-formylcyclohexanone, etc.

As specific examples of the 3-formylcycloalkenones represented by the general formula (2), there can be enumerated 3-formylcyclopentenone, 3-formylcyclohexenone, 5-methyl-3-formylcyclohexenone, 3-formylcycloheptenone, 5,5-dimethyl-3-formylcyclohexenone, 3-formylcyclooctenone, 5-methyl-3-formylcyclooctenone, and 5-phenyl-3-formylcyclohexenone, etc.

Further, if it has the above-described structures, it may be a bicyclic condensed-ring compound having jointly two or more carbon atoms (for example, 4-formylbicyclo[4.4.0]decane-3-en-2-on, etc.).

[(I-3) 3-methylcycloalkanones and 3-methylcycloalkenones]

As starting raw materials for the cycloaliphatic polyisocyanate represented by general formula (1) of the present invention, the above-described compounds represented by the general formula (5) or the general formula (6) are employed.

The compound represented by the general formula (5) is preferably 3-methylcycloalkanones, and the compound represented by general formula (6) is preferably 3-methylcycloalkenones.

In the present invention, 3-formylcycloalkanones represented by the general formula (2) or 3-formylcycloalkenones represented by the general formula (3) which are employed as a substrate in the above-described reductive amination can be prepared by oxidation of the 3-methylcycloalkanones represented by the general formula (5) or 3-methylcycloalkenones represented by the general formula (6) under the presence of catalysts.

Herein, the oxidation under the presence of catalysts includes, for example, a method in which an oxidation by oxygen is carried out under the presence of catalyst such as a metal oxide (selenium dioxide, chromium oxide, bichromic acid, an oxide of copper, silver, and lead, etc.), a salt of naphthenic acid (a salt of cobalt and chromium, etc.), and a vanadium oxide-based catalyst ($V_2O_5$-$SnO_2$, $V_2O_5$-$SnO_2$-$Fe_2O_3$, $V_2O_5$-$Fe_2O_3$), a method in which an oxidation by oxygen is carried out under the presence of at least one kind of metallic salts selected from ruthenium, rhodium, and cobalt which is described in JP-A-58154528 Official Gazette, and a method in which an oxidation by oxygen is carried out under the presence of a heteropolyacid or salts thereof. A preferred method is a method in which the 3-formylcycloalkenones represented by the general formula (3) are obtained by an oxidation of the 3-methylcycloalkenones represented by the general formula (6) under the presence of a catalyst of the heteropolyacid or the combination of the acid with salts thereof.

The above-described heteropolyacid is a condensate of an oxyacid containing at least two different kinds of center ions, and it is also called a heteronuclear condensed acid. The heteropolyacid is composed of, for example, an oxyacid ion (for example, phosphoric acid and silicic acid, etc.) of an element such as P, As, Sn, Si, Ti, and Zr and an oxyacid (for example, vanadic acid, molybdic acid, and tungstic acid, etc.) of an element such as V, Mo, and W, and a variety of heteropolyacids employed as a catalyst can be obtained by the combination thereof.

Anionic composition in the preferred heteropolyacid can be represented by $XM_{12}O_{40}$, and X is an element such as Si and P, M is an element such as Mo, W, and V. As the heteropolyacid having such the composition, for example, there can be exemplified a molybdophosphoric acid, a tungstophosphoric acid, silicomolybdic acid, silicotungstic acid, and phosphovanadomolybdic acid, etc.

Molybdophosphoric acid and phosphovanadomolybdic acid are more preferred and, particularly, phosphovanadomolybdic acid is most preferred.

Phosphovanadomolybdic acid and salts thereof are represented by the following formula.

$$A_{3+n}[PV_nMo_{12-n}O_{40}]$$

(in the formula, A represents a cation of the heteropolyacid, and n is an integer of 1-10.)

The above-described cation of the heteropolyacid represented by A may be, in addition to hydrogen atom, other cations, for example, $NH_4$, an alkali metal (Cs, Rb, K, Na, and Li, etc.), and an alkali earth metal (Ba, Sr, Ca, and Mg, etc.), etc.

It is to be noted that although the heteropolyacid shows a sufficiently high activity as a free heteropolyacid, in the case that the cation of the heteropolyacid is hydrogen atom, it can be at least partially substituted. By partially substituting hydrogen atom in the heteropolyacid, the heteropolyacid becomes insoluble, and stability and heat resistance are elevated, resulting in that it can be changed to a more useful catalyst. As the cation capable of being substituted, it is not particularly limited, for example, there can be exemplified $NH_4$, an alkali metal (Cs, Rb, K, Na, and Li, etc.), and an alkali earth metal (Ba, Sr, Ca, and Mg, etc.), etc. Particularly, in the case that the heteropolyacid is partially substituted with an ammonium cation and cations are composed of $H^+$ and $NH_4^+$, activity and stability are further elevated and, in the case, the proportion of $NH_4$ with respect to hydrogen atom, $NH_4$/H (molar ratio) is preferably 0.1-10, $NH_4$/H (molar ratio) is more preferably 0.2-8, and $NH_4$/H (molar ratio) is most preferably 0.3-5 or so.

Value of n in the above-described formula can be appropriately selected in consideration of an oxidation power and stability, and it is preferably 1-10, more preferably 4-10, most preferably 5-8.

Also, in the case that the cations of the heteropolyacid are composed of H and other cation ($NH_4^+$, etc.), the value of n is often preferably 4-10 or so.

The heteropolyacids or salts thereof may be employed solely or in combination of two or more kinds, and in the case that it is employed solely, it may be employed as a solid catalyst by carrying on a carrier. Like this, by carrying on a carrier, an activity is elevated in the catalyst.

As the carriers for carrying the catalyst components, there are enumerated inorganic carriers such as alumina, silica, silicone carbide, silica-alumina, bentonite, magnesia, titania, vanadia, zirconia, zeolite, diatomaceous earth, and kaolin, and organic carriers such as a styrene-divinylbenzene copolymer, which are commonly employed.

Preferred carriers include porous carriers such as activated carbon, alumina, titania, silicone carbide, silica-alumina, bentonite, and zeolite, and activated carbon is particularly preferred. By the use of activated carbon, a selectivity of methyl group at 3-position in raw materials for reaction can be more elevated in an oxidation reaction. Activated carbon may be powder-or particle-state or fibrous.

Specific surface area in the carrier is not particularly limited, and it is preferably 10-4500 $m^2/g$, and more preferably 50-4000 $m^2/g$ or so. Usually, there is preferably employed one having 100-3000 $m^2/g$ or so. In the case of activated carbon which is a preferred carrier, the specific surface area is preferably 300-4000 $m^2/g$, and more preferably 400-3000 $m^2/g$ or so. Usually, there is often employed activated carbon having 500-2000 $m^2/g$ or so.

Average pore diameter in the activated carbon is preferably 5-200 Angstrom, and more preferably 5-200 Angstrom. Further, pore volume in the activated carbon is preferably 0.1-10 ml/g or so, and more preferably 0.3-5 ml/g or so. Amount of the heteropolyacids or salts thereof to be carried with respect to the carrier can be selected within a range in which a catalytic activity is not deteriorated, and it is usually 0.1-100 parts by weight, preferably 0.5-50 parts by weight, more preferably 5-30 parts by weight or so.

In particular, it is most preferably 5-20 parts by weight or so.

As a method for carrying the heteropolyacids or salts thereof onto or into the above-described carrier, there can be employed commonly-used impregnating methods, coating methods, spraying methods, absorption methods, and sedimentation methods, etc. and, in particular, there can be employed the impregnating methods and absorption methods by which catalyst components can be carried in a uniform and high dispersion state.

In the case of allowing to carry the heteropolyacids or salts thereof, usually, solvents such as water are employed, whereby, a solution of the catalysts can be uniformly carried.

Use amount of the heteropolyacids or salts thereof depends upon kind of the heteropolyacids or salts thereof, and it is usually selected within a range of 0.1-50% by weight based on the heteropolyacids or salts thereof with respect to the raw material represented by the general formulae (5) and (6).

As sources for oxidation of the raw material represented by the general formula (5) or (6) in the present invention, in addition to oxygen and an oxygen-contained gas, there can be also employed a compound capable of producing oxygen. As oxygen, for example, a highly-purified oxygen gas may be also employed and, as the oxygen-contained gas, there may be also employed a mixed gas in which oxygen gas is optionally diluted by a gas which is inert in a reaction such as, for example, nitrogen, helium, argon, and carbon dioxide. In the case of diluting by the inert gas, air can be employed in place of oxygen, resulting in that nitrogen in the air is also employed as an inert gas.

Use amount of oxygen is preferably 0.5-1,000 mol based on 1 mol of the raw material in the reaction, and more preferably an excessively large amount of 1-1,000 mol. Oxidation reaction may be any one of a gas-phase oxidation or a liquid-phase oxidation.

It is to be noted that the reaction may be carried out under the absence of solvents, and it may be also carried out under the presence of solvents which are inert in the reaction. As the solvents, there are enumerated aromatic hydrocarbons such as benzene, toluene, xylene, and ethylbenzene; aliphatic hydrocarbons such as hexane, heptane, and octane; cycloaliphatic hydrocarbons such as cyclohexane; halogenated hydrocarbons such as carbon tetrachloride, chloroform, dichloromethane, and 1,2-dichloroethane; carboxylic acids such as acetic acid, propionic acid, and butylic acid; ketones such as acetone and methylethylketone; esters such as methyl acetate, ethyl acetate, isopropyl acetate, and butyl acetate; ethers such as diethyl ether, dibutyl ether, dimethoxy ethane, dioxane, tetrahydrofran, diethyleneglycol, dimethylether, and 1-methoxy-2-propanol; amides such as N,N-dimethylformamide and N,N-dimethylacetoamide, nitriles such as acetonitrile, propionitrile, and benzonitrile, etc. The solvents may be employed solely or in combination of two or more kinds.

Reaction temperature can be appropriately selected in consideration of reaction rate and selectivity, and it is preferably 30-300°C, more preferably 50-200°C or so.

[(I-4) 3-aminomethylcycloalkylamines]

3-formylcycloalkanones or 3-formylcycloalkenones obtained as described hereinabove are subjected to a reductive amination under the presence of a catalyst to produce 3-aminomethylcycloalkyl amines represented by the general formula (4). It is to be noted that $Y_1$ in the general formula (4) is the same as $Y_1$ in the general formula (1). As specific examples of such the 3-aminomethyl cycloalkylamines, there can be exemplified 3-aminomethyl cyclopentylamine, 3-aminomethylcyclohexylamine, 3-aminomethyl-5-methylcyclohexylamine, 3-aminomethylcycloheptylamine, 3-aminomethyl-5,5-dimethylcyclohexylamine,3-aminomethylcyclooctyl amine, 3-aminomethyl-5-methylcyclooctylamine, and 5-phenyl-3-aminomethylcyclohexylamine, etc. As preferred 3-aminomethyl cycloalkylamines, there can be enumerated 3-aminomethyl-5,5-dimethylcyclohexylamine.

In the above-described cycloaliphatic diamines, amino group and aminomethyl group are situated at a cis-position and a transposition in a cyclohexane ring, and both isomers can be employed as raw materials in the present invention.

It is to be noted that the above-described amines may be substituted with ether bond, sulphone group, carbonyl group, and halogen group, etc. which are a stable group in structure.

In the amines, the aliphatic amines are generally classified into a cycloaliphatic amine which has a cycloaliphatic structure in the molecule and a linear chain aliphatic amine which has a linear chain structure. Although the present invention can be preferably applied to the linear chain aliphatic amine, it can be more preferably applied to the cycloaliphatic amines.

As the amine compounds to be employed in the present invention, there can be preferably enumerated the cycloaliphatic diamines represented by the general formula (4) described hereinabove.

It is industrially valuable to prepare the cycloaliphatic polyisocyanate represented by the general formula (1) having an excellent weatherability which is a final product by employing the cycloaliphatic amine as a raw material.

[(I-5) Reductive amination reaction]

The reductive amination reaction of the above-described substrate is carried out by allowing to react hydrogen and ammonia under the presence of a catalyst.

As the catalysts (hereinafter, occasionally referred to "catalytically active component") to be employed in the above-described reductive amination reaction, there can be exemplified metallic catalysts such as nickel compounds (a reduced nickel and a Raney nickel, etc.), cobalt compounds (a reduced cobalt and a Raney cobalt, etc.), platinum compounds (platinum black and oxidized platinum, etc.), palladium compounds (palladium and palladium black, etc.), rhodium, ruthenium, cobalt-rhenium-molybdenum, copper chromite, and copper-chromium. Of those, preferred catalysts are the nickel compounds, cobalt compounds, platinum compounds, palladium compounds, and cobalt-rhenium-molybdenum, etc.

The catalyst may be employed solely, and it may be employed as a solid catalyst by carrying on a carrier.

As the carrier for carrying the above-described catalytically active components, there are enumerated inorganic carriers such as activated carbon, carbon black, alumina, silica, silicone carbide, silica-alumina, bentonite, magnesia, titania, vanadia, zirconia, zeolite, diatomaceous earth, kaolin, and barium sulphate, and organic carriers such as a styrene-divinylbenzene copolymer, which are commonly employed. Of those, preferred carriers include porous carriers such as activated carbon, alumina, carbon black, silicone carbide, silica-alumina, bentonite, zeolite, and barium sulphate.

Specific surface area in the carrier to be employed is not particularly limited, and it is preferably 0.01-4,500 $m^2/g$, and more preferably 0.1-4,000 $m^2/g$ or so. Usually, there is preferably employed one having 0.2-3,000 $m^2/g$ or so.

Amount of the catalysts to be carried with respect to the carrier can be selected within a range in which a catalytic activity is can be more elevated, and it is usually 0.05-100 parts by weight, preferably 0.1-50 parts by weight, more preferably 0.5-30 parts by weight or so based on 100 parts by weight of the carriers. In particular, it is most preferably 1-20 parts by weight or so.

As a method for carrying the heteropolyacids or salts thereof onto or into the above-described carrier, there can be employed commonly-used impregnating methods, coating methods, spraying methods, absorption methods, and sedimentation methods, etc. and, in particular, there can be employed impregnating methods and absorption methods by which catalytic components can be carried in a uniform and high dispersion state.

Use amount of the catalysts depends upon kind of the heteropolyacids or salts thereof, and it is usually selected within a range of 1-50% by weight based on the substrate in the reaction.

In the above-described reductive amination reaction according to the present invention, there can be any one of hydrogen gas and a hydrogen-containing gas as hydrogen for catalytic reduction. As the former, a highly-purified hydrogen gas may be employed, and as the latter, there may be also employed a mixed gas in which hydrogen gas is optionally diluted by a gas which is inert in a reaction such as, for example, nitrogen, helium, argon, and carbon dioxide.

Hydrogen pressure in the reaction system is usually 1-200 $kgf/cm^2$, preferably 5-150 $kgf/cm^2$, and it ranges in more preferably 10-100 $kgf/cm^2$.

As an ammonia source to be employed for an amination according to the present invention, ammonia alone or an ammonia-contained gas is usually employed, and a compound can be also employed from which ammonia (an ammonium salt) is produced. The ammonia source includes three forms of a gas, a liquid, and a solid, and any forms can be employed. In the case that it is employed as a gas, a highly-purified gas may be also employed, and it may be optionally fed diluting by an inert gas in the reaction system such as, for example, nitrogen, helium, and argon, etc.

The ammonia sources can be employed solely or in combination of two or more kinds depending upon kind of a desired compound.

Regardless of the kind of the above-described ammonia sources, use amount of ammonia according to the present invention is preferably 2-100 times by mol, more preferably 2-50 times by mol with respect to the reaction substrates such as 3-formylcyclo alkanones and 3-formylcycloalkenones.

The above-described reductive amination reaction according to the present invention may be carried out under the absence of solvents, or may be also carried out under the presence of solvents which are inert in the reaction.

As the solvents to be employed in the reductive amination reaction, there can be enumerated, in addition to solvents (an aliphatic hydrocarbon, a cycloaliphatic hydrocarbon, an ester, an amide, and an ether, etc.) employed in an oxidation reaction described hereinafter, alcohols such as methanol, ethanol, propanol, isopropanol, butanol, ethyleneglycol, diethyleneglycol, and 1,4-butanediol, and alcohols and ethers, etc are usually employed for the reductive amination reaction. It is to be noted that the solvents can be also employed in combination of two or more kinds.

Use amount of the above-described solvents is usually 1-100 times by weight or so, preferably 3-50 times by weight or so, and more preferably 5-30 times by weight or so with respect to 3-formylcyclo alkanones and 3-formylcycloalkenones.

Reaction temperature in the reductive amination reaction can be appropriately selected in consideration of reaction rate and selectivity, and it is preferably 30-300°C, more preferably 50-250°C, and most preferably 100-200°C or so.

The above-described reaction can be carried out according to a commonly-used reductive amination reaction. In the case that the solvents are employed, the reaction is carried out by introducing hydrogen into the reaction system which contains 3-formylcyclo alkanones or 3-formylcycloalkenones, ammonia, and the solvents under the presence of catalysts for hydrogenation. In particular, in the case that the reaction is carried out by introducing hydrogen into the reaction system which contains a 3-formylcycloalkene, ammonia, and the solvents, production of by-products can be suppressed.

As described hereinabove, there can be obtained a 3-aminomethylcycloalkylamine which is a cycloaliphatic polyamine.

The cycloaliphatic polyamines represented by the above-described general formula (4) which are produced in the reaction can be readily refined solely or in combination of commonly-used means for separation, for example, a variety of separation means such as filtration, concentration, distillation, extraction, crystallization, recrystallization, and a column chromatography.

[(I-6) Conversion of an amine into an isocyanate]

The 3-aminomethylcycloalkylamines represented by the general formula (4) are converted into cycloaliphatic polyisocyanates represented by the general formula (1) by converting amino group into isocyanate group through publicly-known methods.

Many methods are proposed for converting amino group into isocyanate group, and the cycloaliphatic polyisocyanates represented by the general formula (1) according to the present invention can be prepared by using the methods.

[(I-6-1) Conversion into an isocyanate by a phosgene method]

Of the publicly-known methods, a method by the use of phosgene is a method which is often employed for industrial preparation of isocyanates even at present time. It has a high practicability in view of many existing facilities to be utilized and a high yield in a process.

As specific examples, there can be enumerated a method in which polyisocyanates are prepared by an addition reaction of phosgene to polyamines at low temperatures in a solvent (for example, chlorobenzenes) not having hydrogen atoms, and then by dehydrochlorination and further dehydrochlorination at high temperatures according to the following process.

$$RNH_2 + COCl_2 \rightarrow RNHCOCl + HCl \rightarrow RN{=}C{=}O + HCl$$

[(I-6-2) Conversion into an isocyanate by a carbamate method]

As other specific examples, there can be enumerated a method (it is called a carbamate method or a urethane method) in which polyisocyanates are prepared by converting the polyamines into corresponding carbamates and by removing alcohols according to the following process.

First stage reaction

$$RNH_2 + R'OC(=O)OR' \rightarrow RNHCOOR' + R'OH$$

Second stage reaction

$$RNHCOOR' \rightarrow RN=C=O + R'OH$$

Polycarbamates represented by the general formula (7) in the present invention are obtained as following compounds depending upon polyamines to be employed.

There can be enumerated 3-methoxycarbonylaminomethyl-1-methoxycarbonyl aminocyclopentane, 3-methoxycarbonylaminomethyl-1-methoxycarbonyl aminocyclohexane, 3-methoxycarbonylaminomethyl-5-methyl-1-methoxycarbonylaminocyclohexane, 3-methoxycarbonyl aminomethyl-1-methoxycarbonylaminocycloheptane, 3-methoxycarbonylaminomethyl-5,5-dimethyl-1-methoxycarbonyl aminocyclohexane, 3-methoxycarbonylaminomethyl-5-methyl-1-methoxycarbonylamino cyclooctane, and 5-phenyl-3-methoxycarbonyl aminomethyl-1-methoxycarbonyl aminocyclohexane, etc.

Herein, as a method for preparing a carbamate, there are proposed a method in which a carbonate such as dimethylcarbonate is allowed to react with amines, and a method in which urea and an alcohol are allowed to react with amines, and both of those can be applied.

Further, in the above-described processes, as a method for preparing an isocyanate by dealcoholation of a carbamate, there is preferred a method in which a carbamate is thermally decomposed under the presence of metallic acidic catalysts, and then separating a by-produced alcohol by distillation, etc. to recollect an isocyanate which is a desired product.

Yield is high in the carbamate-through process, and it is excellent as a process in which phosgene having a high toxicity is not employed.

The polyisocyanates obtained by the above-described various methods can be refined until a desired purity from a reaction crude liquid by commonly-used means for separation, for example, a variety of separation means such as filtration, concentration, distillation, extraction, crystallization, recrystallization, and a column separation means, or the combination thereof.

[(II) A process for the preparation of cycloaliphatic polyisocyanates by the carbamate-through process and cycloaliphatic polyisocyanates having the chlorine content of not more than 10 ppm]

Hereinafter, there is illustrated in detail a process for the preparation of cycloaliphatic polyisocyanates by the carbamate-through process and cycloaliphatic polyisocyanates having the chlorine content of not more than 10 ppm.

[(II-1) Conversion into an isocyanate by a carbamate-through process]

As roughly mentioned in the item of [(I-6-2) Conversion into an isocyanate by a carbamate method], the above-described purpose can be achieved by a method in which an amine is allowed to react with a dialkyl carbonate to prepare a carbamate compound, followed by thermally decomposing it to obtain a polyisocyanate.

Recently, a preparation technology was disclosed (JP-A-64085956) in which a diamine is converted into an isocyanate by dimethylcarbonate, and it is applied as a method for the preparation of the cycloaliphatic polyisocyanates to be employed in the present invention.

The technology disclosed in the JP-A-64085956 Official Gazette is composed of two stage reactions as roughly mentioned in the item of [(I-6-2) Conversion into an isocyanate by a carbamate method].

First stage reaction is a reaction in which a dialkylcarbonate compound such as dimethylcarbonate is allowed to react with a cycloaliphatic polyamine in a reaction molar ratio of 2-50 (molar ratio of a dialkylcarbonate/amino group = 1-25) to prepare a corresponding cycloaliphatic carbamate.

In particular, there is preferred the molar ratio of a dialkylcarbonate/cycloaliphatic polyamine of not less than 4.

Because, yield of a cycloaliphatic dilcarbamate represented by the general formula (7) is necessarily improved by suppression of a dimerization reaction in the molar ratio of a dialkylcarbonate/cycloaliphatic diamine of 2-50. When

the use amount of the dialkylcarbonate exceeds the molar ratio of 50, it becomes not effective.

It is preferred to adjust water content in the polyamine to be employed to less than 1 ppm. Because it makes lower an activity of the catalysts as well as water content in the dialkylcarbonate described hereinafter.

It is preferred to adjust the water content in the dialkylcarbonate to be employed to less than 0.2%. Because water in the dialkylcarbonate reacts with the catalysts, and change to a hydroxide of a metal. The hydroxide of a metal does not show a catalytic effect, resulting in that the use amount of the catalysts must be increased.

A dialkylcarbonate prepared by usual methods has a property of relatively dissolving water. Accordingly, it includes a high risk of water mingling.

As examples of basic materials which are a catalyst for employing in the first stage, which are a alcolate of an alkali metal or an alkali earth metal, there can be enumerated a methylate, an ethylate, and tertiary butylate of lithium, sodium, potassium, calcium, and barium. The basic materials can be in a state of a solid or a solution.

Of the above-described materials, sodium methylate is particularly preferred because of readily obtaining and economy.

Use amount of sodium methylate is does not economically affect, and it does always not require to be recirculated.

Accordingly, a plant is simple.

Further, sodium methylate is commercially supplied as a solution of methanol, and it is easy in handling.

Use amount of the alcolate of an alkali metal or an alkali earth metal is decided depending upon activity thereof so that the reaction is terminated within a practical time of period. In the case of sodium methylate, the reaction progresses in the amount of 0.001-5% by weight, preferably 0.1-3% by weight in a crude reaction liquid.

Reaction temperature can be practically selected within a range from 0°C to boiling point. In a low temperature, reaction is slow, and in a low temperature, since boiling of methanol by-produced becomes vigorous, it is preferably selected in a range from 30°C to 80°C.

Amine compounds are preferably added dropwise into dimethylcarbonate in view of controlling of reaction heat.

In the case that the use amount of sodium methylate is less than 0.001% by weight, the reaction rate is slow and, in the case that it is more than 5% by weight, precipitation of the catalysts becomes problematic, and it also becomes disadvantageous in economy.

In the case that starting raw materials are solid, and in the case of preventing precipitation of a urethane compound, solvents are employed without any problems, there can be employed a solvent, for example, such as methanol, ethanol, tetrahydrofran, dioxane, benzene, and toluene which are inert to the starting raw materials and a product.

Kind and amount of the solvents are selected depending upon reaction conditions in which starting raw materials and a product are dissolved. When the use amount of the solvents is much, that is, dilution ratio is high, the reaction slowly progresses, resulting in being disadvantageous. The use amount of the solvents is advantageously limited to a minimum amount to be required for dissolving the starting raw materials and a product. It is desirably employed in an amount of 1-10 times based on the cycloaliphatic dicarbamate produced.

In the case that the molar ratio of a dialkylcarbonate/aliphatic polyamine is approximately 2 in the starting raw materials, since the concentration of the cycloaliphatic dicarbamate produced becomes high in a reaction crude liquid, in the case of the cycloaliphatic dicarbamate having a high crystallinity, there must be selected a solvent having a high dissolving power for the purpose of avoiding a risk of crystallization.

Further, there is preferably employed a solvent having a boiling point of not less than 10°C lower than the cycloaliphatic dicarbamate produced, whereby, distillation for refining becomes easy and it is advantageous in view of economy.

As a method for mixing the starting raw materials, particularly, in the case of a batchwise reaction, it is preferred that the catalyst is continuously added with the progress of the first stage reaction or it is added by an intermittently separating method of a plurality of times, whereby, the use amount of the catalyst can be preferably decreased to 1/2-1/3 compared to a method to be added in the lump.

Although the reason is not clear, it was found out that the use amount of the catalyst can be preferably decreased by a continuous addition or an intermittent addition.

In the case that feeding rate is quick in the cycloaliphatic polyamine and the catalyst, since boiling of methanol by-produced becomes vigorous, it is required that the feeding rate of the catalyst is controlled together with the reaction temperature.

Further, in the case that the first stage reaction is continuously carried out, the cycloaliphatic polyamine and the catalyst can be also supplied not only from an inlet of a reaction apparatus but also from a middle position of the reaction apparatus.

For example, in the case that a reaction crude liquid is flown in series into a plurality of completely-mixable vessels, there can be applied a method that the catalyst is separately supplied into respective vessels.

Although the method can be sufficiently carried out at an ordinary pressure without compressing, it can be also appropriately carried out even at a compressed condition generated by a pressure loss derived from a structure of an apparatus.

A crude liquid of the cycloaliphatic dicarbamate produced can be refined until a desired purity by conventional refining methods such as distillation, crystallization, water washing, and reprecipitation.

Above all, excessively added acid components are removed by water-washing after neutralization of alkali components derived from a metal alcolate which is a basic catalyst using phosphoric acid, sulfuric acid, formic acid, oxalic acid, and acetic acid, etc.

When the basic catalyst is heated together with the cycloaliphatic dicarbamate, since it further changes the cycloaliphatic dicarbamate to compounds having high-boiling point which are undesired products, a neutralization step is required after a first stage reaction.

Salts produced in the neutralization step can be removed by common methods such as water-washing, filtration, and centrifugal separation in combination with removal of an alcohol.

The cycloaliphatic dicarbamate is taken out by further refining until a desired purity by common refining methods such as flash evaporation, distillation, crystallization, water-washing, and reprecipitation.

In a second stage reaction, the cycloaliphatic dicarbamate is thermally decomposed in an inert solvent having a high boiling point such as dibenzyl toluene, partially-hydrogenated triphenyl, and m-terphenyl under the presence of at least one catalysts selected from the group of metals, inorganic compounds, and organic compounds such as manganese, molybdenum, tungsten, and zinc at reduced pressures, and there is caused an elimination of an alcohol to obtain a corresponding cycloaliphatic polyisocyanate.

A reaction formula is shown below.

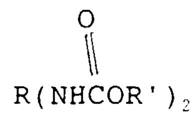

Cycloaliphatic dicarbamate C

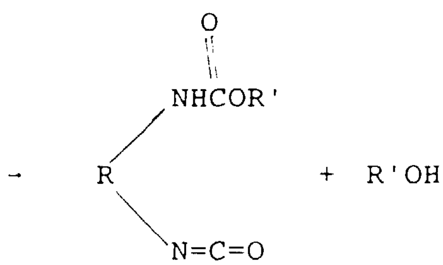

Cycloaliphatic monoisocyanate

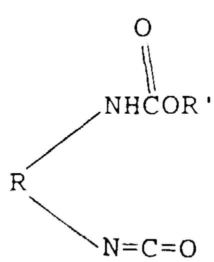

Cycloaliphatic monoisocyanate

$\rightarrow R(N=C=O)_2 + R'OH$

Cycloaliphatic diisocyanate
(in the above formulae, R represents a divalent cycloaliphatic structure)

By the use of the catalysts, a quicker reaction rate is obtained, and concentration of the cycloaliphatic polyisocyanate is maintained in a low level by carrying out the reaction in a solvent at reduced pressures in which the cycloaliphatic polyisocyanate is distilled out, whereby, there are controlled dimerization and trimerization of an isocyanate group, and the following addition reaction of NH group in a carbamate bond with an isocyanate group, resulting in that a high yield can be attained.

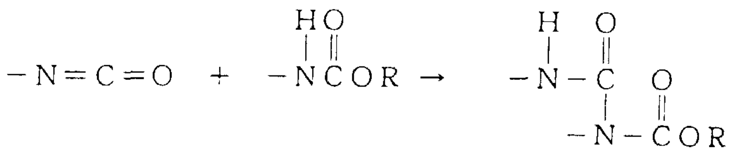

As compounds to be employed as a catalyst, there can be exemplified metallic manganese, manganese oxide (MnO or $Mn_2O_3$), manganese chloride, manganese sulphate, manganese phosphate, manganese borate, manganese carbonate, manganese acetate, manganese naphthate, manganese (II) acetylacetonate, manganese (III) acetylacetonate, metal molybdenum, molybdenum trioxide, molybdenum acetylacetonate ($MoO_2(acac)_2$), molybdenum dioxide, metal tungsten, tungsten hexacarbonyl, tungstic anhydride, tungstic acid, and zinc, etc.

These can be employed as a water-contained salt or an anhydride. Manganese chloride, manganese sulphate, manganese acetate, and manganese naphthate are particularly preferred because of being capable of easy obtaining, a low price, and a high activity.

Particularly, manganese acetate is preferred because of a sufficient activity even at a low concentration in the reaction crude liquid.

Use amount of the catalysts is appropriately decided depending upon reactivity of starting raw materials, temperature, the kind and amount of the catalysts.

In the case that the use amount of the catalysts is excessively less, reaction rate is slow and, in the case of being excessively much, there tends to increase the amount of by-produced compounds having high boiling point. Usually, it is most preferred in a range of 0.0005-5% by weight.

Reaction temperature is preferred in a range of 150-300°C. In the case that it is lower than 150°C, the production of the cycloaliphatic polyisocyanate is unpractically slow and, in the case that it is higher than 300°C, it is difficult to industrially carry out, resulting in becoming disadvantageous.

It is required that solvents are inert to the cycloaliphatic polyisocyanate which is a desired compound and the cycloaliphatic dicarbamate, and which are selected from an aliphatic compound, an aromatic compound, an alkylated aromatic compound, and an ether compound. The solvents may contain an inert group such as a halogen group.

Also, there is preferred a solvent being capable of readily separating from the cycloaliphatic polyisocyanate which is a desired compound. There is preferred a solvent having a boiling point being apart from the cycloaliphatic diisocyanate in view of capability of readily refining by distillation.

There is preferred a solvent having a higher boiling point than the cycloaliphatic polyisocyanate. In the case that there is employed a solvent having a lower boiling point than the cycloaliphatic polyisocyanate, since it is distilled out together with the cycloaliphatic polyisocyanate, a process becomes unpreferably complicated from a viewpoint of practical use.

There is particularly preferred a solvent having 10°C higher boiling point than the cycloaliphatic polyisocyanate produced because of being capable of readily refining by distillation in a further stage.

Further, since the by-products having high boiling point increase with a lapse of time in the solvents, there is preferred a solvent having a boiling point at which can be industrially regenerated.

As preferred solvents, there are exemplified o-terphenyl, m-terphenyl, p-terphenyl, a mixed diphenyl benzene, a partially-hydrogenated triphenyl, dibenzyl benzene, dibenzyl toluene, biphenyl, phenylether, phenylcyclohexane, hexadecane, teradecane, octadecane, eicosane, benzylether, and tetramethylene sulfone, etc.

Although there should be selected a preferred solvent depending upon the cycloaliphatic polyisocyanate, dibenzyl toluene, a partially-hydrogenated triphenyl, and m-terphenyl is particularly preferred.

Reaction is carried out under a reduced pressure at which the cycloaliphatic polyisocyanate produced is distilled out from a reaction system, whereby, there is obtained an effect that concentration of the cycloaliphatic polyisocyanate in the reaction system is maintained in a low level, resulting in that a high reaction yield can be attained. Particularly, the effect is efficiently attained by by carrying out under boiling of the solvent. From the viewpoint, the reaction is preferably carried out at temperatures and a degree of reduced pressure at which the solvents are boiled.

In the case that the degree of reduced pressure is excessively high, it becomes difficult to recollect the alcohols

by-produced, and the degree of reduced pressure is preferably 1-700 Torr in order to avoid disadvantages in view of equipments and utilities.

The cycloaliphatic polycarbamate which is a starting raw material in the reaction by thermal decomposition, a solvent, and a catalyst are supplied into a reaction system. A continuous reaction is advantageous in which the cycloaliphatic dicarbamate is supplied into the solvent boiled containing the catalyst.

An alcohol by-produced in the reaction and an isocyanate compound are introduced from a reaction vessel to a condenser in the form of a gas, and the isocyanate compound alone can be condensed to excellently refine.

The solvents in the reaction system are continuously taken out together with the by-produced compounds having high boiling point in the solvents, and solvents not containing the by-produced compounds having high boiling point are supplied into the reaction system.

As described hereinafter, it is advantageous to refine and reuse the solvents taken out.

Since the catalysts are lost together with the solvents taken out, the same amount of the catalysts are continuously supplied as the catalysts lost to constantly maintain the concentration of the catalysts.

The cycloaliphatic dicarbamate and the solvents may be supplied into the reaction system after being in advance mixed.

In the case that manganese acetate is employed as a catalyst, it was conventionally employed as a methanol solution by applying a high solubility of manganese acetate to methanol.

A main purpose for dissolving, when a catalyst is a solid or a viscous substance, exists in easy-handling by changing to a solution. However, since temperature in the reaction system is higher than the boiling point of methanol, it is observed that catalysts deposit by evaporation of methanol.

If being added by dissolving the methanol solution of the catalysts into the cycloaliphatic polyisocyanate which is a desired compound, since the cycloaliphatic polyisocyanate has a high boiling point, it is effective for preventing the deposition of the catalysts.

The catalysts are prepared according to the following steps.

First of all, the catalysts are dissolved in methanol to obtain a solution of the catalysts. Mixing ratio of the catalysts with respect to methanol is preferably a ratio in which a solution becomes a uniform solution having a low viscosity, or a larger amount of methanol is employed. However, the use of an excessively larger amount of methanol is uneconomical.

The weight ratio of the catalysts with respect to methanol is selected depending upon the kind of the catalysts, and it is preferably 0.2-1,000 times by weight based on the weight of the catalysts.

At that time, there must be drawn an attention so that methanol does not return to the cycloaliphatic dicarbamate by the reaction of methanol with the cycloaliphatic polyisocyanate.

Usually, a compound having a carbamate group has a high melting point and high viscosity than a compound having an isocyanate group.

That is, there is preferably selected a ratio in which viscosity does not increase, even a reaction of the cycloaliphatic polyisocyanate with methanol completely terminates.

Further, even methanol exists more excessively than the cycloaliphatic polyisocyanate, since viscosity is low owing to a dilution effect by methanol, the reaction can be carried out.

However, when methanol is fed into a reaction system at a composition ratio in which methanol exists more excessively, a means for heating, etc. is occasionally required because of a vigorous vaporization of methanol.

There is unexplained a reason that a catalyst which does not dissolve in the cycloaliphatic polyisocyanate can be mixed with the cycloaliphatic polyisocyanate after being dissolved into methanol, and a uniform solution is obtained. It is presumed that it possibly depends upon a polar carbamate bond produced and solubilizing by an coordination on the catalyst. A reason why methanol is preferred depends upon that methanol is produced in the thermal decomposition and a refining system does not become complicated.

If a solvent in a reaction dissolves a necessary amount of a catalyst, although the catalyst can be supplied as a solution, since the solvent in a reaction is usually nonpolar and dissolving power is often insufficient, the above-mentioned method is effective. A reason why the mixing order in the solution is as described above depends upon that the manganese compound does not dissolve in the cycloaliphatic polyisocyanate and readily dissolves in methanol.

Further, in a distillation column to be employed, the stage number is selected according to physical properties in of the cycloaliphatic dicarbamate to be decomposed, the cycloaliphatic polyisocyanate to be produced, and solvents. Regardless of the stage number, the present method is effective. Actually, the reaction can be preferably carried out by a distillation column corresponding to 1-100 stages. Plant cost becomes expensive in a distillation column exceeding 100 stages. A catalyst solution can be supplied at any positions in a distillation column, and an effect is shown compared to be supplied into a reaction vessel.

However, in the case that the catalyst solution is supplied at an excessively low position in a distillation column, a dicarbamate cannot be sufficiently decomposed because a reaction zone is formed at a position lower than a supplying position of the catalyst solution. On the other hand, in the case that the catalyst solution is supplied at an excessively

higher position in a distillation column, since monoisocyanate in the catalyst solution is distilled out, it becomes rather disadvantageous. Accordingly, there is selected a most preferred position to be well-balanced according to a cycloaliphatic diisocyanate to be produced.

Further, by-products having a high boiling point are produced in the catalyst solution, and the by-products cause an addition reaction with isocyanate group in the cycloaliphatic polisocyanate which is a final product, whereby, there lowers yield in the cycloaliphatic polyisocyanate.

As a method for preventing a yield decline, a crude liquid mixture in the reaction vessel is continuously discharged to flash-evaporate, and a mixed gas in which solvents having a high boiling point are rich is condensed together with removal of by-products having a high boiling point which are residual products in a bottom of a flash-evaporator, and there is recirculated a mixed liquid composed of condensed solvents, the unreacted cycloaliphatic dicarbamate, and the cycloaliphatic polyisocyanate which is a final product into the reaction vessel.

The by-produced compounds having high boiling point may be also removed by distillation using a distillation column in place of flash evaporation. In the distillation, since the catalysts are also lost by being taken out together with the by-produced compounds having high boiling point, a corresponding amount is additionally fed into the reaction system.

Although the catalysts can be also employed again after being recovered, since the catalysts to be employed in the present invention are low in price and the use amount is also slight, cost does not increase by no recovery of the catalysts.

It is to be noted that yield of the cycloaliphatic polyisocyanate remarkably is improved by the use of a catalytic accelerator together with the catalysts in the thermal decomposition. As the catalytic accelerator to be employed, triesters of phosphite are particularly preferred.

As specific examples of the triester of phosphite, there are exemplified a trialkylester of phosphite such as triethyl of phosphite and tributyl of phosphite, and a triarylester of phosphite such as triphenyl of phosphite and tritolyl of phosphite.

Of those, esters having high boiling point such as triethyl of phosphite are preferred because of a little amount of loss by volatilization during the reaction.

In the case that the triesters of phosphite are employed, the use amount is 0.01-10,000 times by weight with respect to the manganese compounds. In the case that it is less than 0.01 times by weight, an effect by addition is not shown and, contrarily, even in the case of exceeding 10,000 times by weight, the effect is not unpreferably improved.

As described hereinabove, there can be obtained the cycloaliphatic polyisocyanate having the content of chlorine or chlorine compounds of not more than 10 ppm.

Dimethyl carbonate having a low content of chlorine must be prepared not through phosgene, and dimethylcarbonate prepared using carbon monoxide and methanol is low in price and preferred as starting raw materials . Commercially supplied dimethylcarbonate can be employed without refining, and optionally with refining.

[(II-2) Dialkyl carbonate]

The cycloaliphatic polyisocyanate of the present invention is not particularly limited, and a polyisocyanate is preferred which is produced by isocyanation through the reaction of the dialkyl carbonate typified by dimethylcarbonate with the cycloaliphatic polyamine owing to a small amount of chlorine compounds.

Although the dialkyl carbonate itself has been once prepared by using phosgene as a raw material, nowadays, a new technology for industrial production is being established.

[(II-2-1) Dialkyl carbonate by a phosgene method]

In the case that a polyisocyanate compound is prepared by using a dialkyl carbonate prepared using phosgene as a starting raw material, the amount of chlorine compounds in a final product fluctuates in a range of 10-50 ppm. As described above, fluctuation in the amount of chlorine compounds causes a difficult control in the use amount of catalysts such as protonic acids. That is, the content of chlorine compounds must be in advance measured in order to decide the use amount of catalysts.

It is to be noted that a process for the preparation of a dialkyl carbonate using phosgene is disclosed in JP-B-87085350, JP-A-62197639, and JP-A-61118349 Official Gazettes, etc.

[(II-2-2) Dialkyl carbonate by a nonphosgene method]

On the other hand, in a polyisocyanate compound prepared by thermal decomposition of a carbamate compound which is prepared using a dialkyl carbonate prepared by using phosgene, since the content of chlorine compounds is a minor amount of not more than 1 ppm, and it is constant, the content of chlorine compounds does not need

to be in advance measured.

The phrase "without using phosgene" often mentioned in the present invention means that phosgene is not employed even in the preparation step of the dialkyl carbonate.

Since a preferred cycloaliphatic polyisocyanate employed in the second aspect of the present invention is prepared by a method in which phosgene is not employed at all, the chlorine compounds substantially derived from phosgene are not contained, and as a result, an adverse affection to physical properties caused by the chlorine compounds is exceedingly minor, resulting in that the purpose of the present invention can be achieved.

[(II-2-2-1) Dialkyl carbonate by an alcohol-carbon monoxide method]

A method for preparing the dialkylcarbonate "without using phosgene" is disclosed in JP-A-63057522, JP-A-63072650, JP-A-63072651, JP-A-01287062, JP-B-85058739, JP-B-81008020, JP-B-85023662, JP-B-86008816, JP-B-86043338, JP-B-88038018, and JP-B-87008113 Official Gazettes, etc. Starting materials in the methods are carbon monoxide, an alcohol, and oxygen, and it is a method in which a reaction is carried out under ordinary or compressed pressures.

Hereinafter, a method in the JP-A-01287062 Official Gazette is typically illustrated.

Starting materials in the method are carbon monoxide, an alcohol, and oxygen, and it is a method in which a reaction is carried out under ordinary or compressed pressures.

Catalysts to be employed are a divalent copper salt and, specifically, copper salts of a carboxylic acid such as copper acetate, copper pivarate, and copper benzoate, copper hydrobromide, copper salts of a weak acid such as copper carbonate, and copper salts of phenols, halogenated compounds such as cupric chloride and cupric bromide, etc.

Use amount of the divalent copper salts to be employed is 1-3,000 mmol, preferably 10-1,000 mmol based on 1 liter of an alcohol.

The above-described catalysts are preferably employed together with a halogenated compound of alkali earth metals.

As specific examples of the alkali earth metals, there are exemplified beryllium, magnesium, calcium, and barium, and there are employed chlorides, bromides, and acetic acid salts thereof, etc.

The halogenated compound of alkali earth metals is preferably employed in an amount of 1/10-10 times by mol based on the divalent copper salts, and it is preferably employed in an amount ranging in 1/2-2 as atomic ratio (halogen/copper) of halogen with respect to copper in a catalyst system.

As the catalysts to be employed together, in addition to the above-described divalent copper salts, there are exemplified platinum-group compounds such as ruthenium compounds, palladium compounds, and rhodium compounds. These are employed in the form of a halogenated compound, a salt of an acetic acid, and a salt of a nitric acid. However, in the case that the halogenated compound is employed as a catalyst, the concentration of halogens becomes somewhat higher in the dialkyl carbonate obtained.

However, since the halogenated compound is removed in respective steps, it becomes not almost present in the polyisocyanate compounds.

In the case that the platinum-group compounds are employed together, the concentration thereof is not more than equal mol, preferably not more than 1/10 based on the divalent copper salts.

As the alcohols which are a starting raw material in the process, there can be an aliphatic alcohol such as methanol and ethanol, and an aromatic alcohol can be also employed. Of the variety of alcohols, methanol is most preferred.

In the case that methanol is employed, the dialkyl carbonate obtained is dimethyl carbonate. Starting raw materials other than methanol in the process are carbon monoxide and oxygen, and those do not always require a high purity. Those may be also diluted by a gas which is inert in the reaction such as nitrogen, argon, and carbon dioxide.

The reaction is carried out at 1-100 atm, and in the case that the reaction system is diluted by the inert gas, partial pressure of carbon monoxide and partial pressure of oxygen are preferably adjusted to 0.1-10 atm, respectively. The reaction temperature preferably ranges in 20-250°C.

In the dialkyl carbonate prepared as described hereinabove, chlorine content is at most 15 ppm even in the case that a chlorine compound (a copper halide, etc.) is employed as a catalyst, and as described hereinafter, in the case that a cycloaliphatic polyisocyanate compound is prepared by a reaction of the dialkyl carbonate with a cycloaliphatic polyamine illustrated hereinafter, the chlorine content in the polyisocyanate compound becomes further lower.

It is to be noted that the chlorine content in a dialkyl carbonate prepared by the use of phosgene is known to be 150-600 ppm.

In the case that a cycloaliphatic polyisocyanate compound is prepared by the reaction of the dialkyl carbonate having such a chlorine content with a polyamine, the chlorine content in the cycloaliphatic polyisocyanate compound

is 15-60 ppm, and approximately 10% of the amount chlorine contained in the dialkyl carbonate is remained in the cycloaliphatic polyisocyanate compound.

[(II-2-2-2) Dialkyl carbonate by an alkylene oxide-carbon dioxide-methanol method]

As another method for preparing the dialkyl carbonate without using phosgene, there is an alkylene oxide-carbon dioxide-methanol method. The method is composed of two steps.

First of all, in a first step, an alkylene carbonate is prepared by an alkylene oxide and carbon dioxide which are starting raw materials, and then an alcohol such as methanol is allowed to react to obtain the dialkyl carbonate, for example, dimethyl carbonate.

A method in relation to the first step is disclosed in, for example, JP-B-73027314, JP-A-51013720, JP-A-51019722, JP-A-51019723, JP-A-51118763, and JP-A-59128382 Official Gazettes, etc.

In a second step, an alkylene carbonate prepared in the first step is allowed to react with an alcohol to obtain a dialkyl carbonate.

A method in relation to the second step is disclosed in, for example, JP-B-85022697, JP-B-85022698, JP-B-86004381, JP-B-84028542, JP-B-81040708, JP-B-86016267, JP-B-85027658, JP-B-84 028542, JP-A-03044353, and JP-A-03044354 Official Gazettes, etc.

[(II-2-2-3) Dialkyl carbonate by other methods]

In addition, as other methods for preparing the dialkyl carbonate without using phosgene through steps, although not being commercially put into practice, the following prior technologies are also disclosed. For example, those are JP-B-81008020 and JP-B-86008816, etc.

In the technologies, although starting raw materials are carbon monoxide, an alcohol, and oxygen, combination in catalysts to be employed is somewhat different from the technologies described in the above-mentioned JP Official Gazettes.

[(III) Cycloaliphatic polyisocyanate composition containing a protonic acid]

Hereinafter, there is illustrated a cycloaliphatic polyisocyanate composition primarily containing the cycloaliphatic polyisocyanate obtained as described hereinabove, and which contains a protonic acid.

There are not particularly limited the cycloaliphatic polyisocyanates to be employed for preparing the composition of the present invention, and those are the cycloaliphatic polyisocyanate obtained as described hereinabove. There is preferred a cycloaliphatic polyisocyanate prepared by an isocyanation reaction of the cycloaliphatic polyamine with a dialkyl carbonate typified by dimethyl carbonate, that is, a cycloaliphatic polyisocyanate having a low content of the chlorine compounds because of a large effect by the addition of the protonic acid.

The cycloaliphatic polyisocyanate to be employed for the composition of the present invention is a compound represented by the above-described general formula (1), and although the preparation process thereof is described hereinabove, the preparation process for the dialkyl carbonate and the cycloaliphatic dicarbamate is not limited. However, in the cycloaliphatic polyisocyanate, the content of the chlorine compounds is preferably low because of a large effect by the addition of the protonic acid.

For example, there is disclosed a method (JP-B-76033095) in which dimethyl carbonate is allowed to react with an amine using a Lewis acid as a catalyst, and it can be applied for a first step reaction of the carbamate method.

Further, there can be also employed a cycloaliphatic polyisocyanate obtained by a method (JP-A-59202352 and JP-A-59202353) for thermally decomposing in a gas phase and a method (JP-B-81045736) in a liquid phase without any problems, as a second step reaction of the carbamate method.

In the present invention, the protonic acid to be mixed with the cycloaliphatic polyisocyanate is a generic name of Bronsted acids which discharge a proton according to pKa which is a inherent constant in dissociation of an acid.

As examples of the protonic acid to be mixed with the cycloaliphatic polyisocyanate in the present invention, there can be exemplified a mineral acid such as nitric acid, sulfuric acid, phosphoric acid, and phosphorous acid; saturated organic acids having a carbon number of 1-18 such as formic acid, acetic acid, propionic acid, butyric acid, caproic acid, caprylic acid, capric acid, lauric acid, mirystic acid, palmitic acid, and stearic acid; unsaturated organic acids having a carbon number of 3-18 such as acrylic acid, methacrylic acid, vinyl acetic acid, sorbic acid, oleic acid, linolic acid, and linolenic acid and a dimer acid thereof; aromatic acids such as cinnamic acid, benzoic acid, and salicylic acid with the alkyl or alkenyl substituent having a carbon number of 1-18, polybasic acids having a carbon number of 2-18 such as oxalic acid, adipic acid, malonic acid, palmitic acid, phthalic acid, isophthalic acid, trimellitic acid, and pyromellitic acid and partial esters thereof; alkylsulfuric acids having a carbon number of 1-18 such as laurylsulfuric acid, p-toluene sulfonic acid, dodecylbenzene sulfonic acid, alkylphenyl sulfuric acids having a carbon number of 6-24;

phosphine acids and phosphone acids with an alkyl group having a carbon number of 1-18, an alkenyl group having a carbon number of 2-18, and an alkylphenyl group having a carbon number of 6-24, phosphites and phosphates with an alkyl group having a carbon number of 1-18, an alkenyl group having a carbon number of 2-18, and an alkylphenyl group having a carbon number of 6-24 such as dimethylphosphite, diethylphosphite, diphenylphosphite, dimethylphosphate, diethylphosphate, diphenylphosphate, and 2-ethylhexylphosphate, etc.

Of the protonic acids for accelerating the conversion of a polyisocyanate into a polyurethane, the saturated organic acids are particularly useful because those are almost a liquid state or can be readily liquified by heating, and those have a high solubility to the cycloaliphatic polyisocyanate, and further, those can be readily obtained owing to a low price.

The amount of the protonic acids, which are added to the cycloaliphatic polyisocyanate prepared from a cycloaliphatic polyamine and a dialkylcarbonate prepared without substantially using phosgene through preparation steps, is preferably $1x10^{-7}$-$1x10^{-1}$ equivalent, and more preferably $1x10^{-6}$-$1x10^{-2}$ equivalent based on 1 equivalent of the NCO group. Total amount of the acids containing the protonic acids and Lewis acids is preferably $1x10^{-8}$-$1x10^{-1}$ equivalent based on 1 equivalent of the NCO group.

In the case that the amount of the protonic acids to be added is less than $1x10^{-7}$ based on 1 equivalent of the NCO group, there is only obtained a composition having a low effect for accelerating the conversion of a polyisocyanate into a polyurethane.

On the other hand, in the case that the amount of the protonic acids to be added is more than $1x10^{-1}$ based on 1 equivalent of the NCO group, additional effects will be not only shown but also the protonic acids will react with a polyisocyanate to lower the quality and properties of the polyurethane.

In the present invention, the addition of the protonic acid to be mixed in order to accelerate the conversion of the cycloaliphatic polyisocyanate prepared from a cycloaliphatic polyamine and a dialkylcarbonate may be carried out either by previously adding a fixed amount of a protonic acid to a cycloaliphatic polyisocyanate to form a composition having an enhanced reactivity or by feeding a protonic acid and a cycloaliphatic polyisocyanate into a reactor simultaneously like in the case of other catalysts.

In the case, it is important that the water content of the protonic acid to be mixed is previously lowered as small as possible. If a large amount of water is mixed into the cycloaliphatic polyisocyanate, it will be converted into urea or an allophanate to bring out a lowering in the quality.

It is to be noted that the dialkylcarbonate illustrated in the present invention includes dimethyl carbonate, diethyl carbonate, and diallyl carbonate, etc. Further, diphenyl carbonate can be also employed. Of those, dimethyl carbonate is preferred.

[(IV) Cycloaliphatic polyisocyanate composition containing a Lewis acid]

Hereinafter, there is illustrated a cycloaliphatic polyisocyanate composition primarily containing the cycloaliphatic polyisocyanate obtained without using phosgene through the steps as described hereinabove, and which contains a Lewis acid.

A composition primarily containing the cycloaliphatic polyisocyanate of the present invention can be prepared by mixing the the cycloaliphatic polyisocyanate with a fixed amount of Lewis acids described hereinafter.

The cycloaliphatic polyisocyanate to be employed in the present invention is the same as illustrated in the first aspect of the present invention, and the cycloaliphatic polyisocyanate illustrated in the second aspect of the present invention are employed in uses in which mingling of chlorine components becomes problematic, which are prepared without using phosgene through all steps.

As the Lewis acids to be mixed, there can be exemplified halogenated compounds and/or salts of manganese, tin, zinc, lead, iron, magnesium, antimony, titanium, boron, and aluminum, etc.; salts of manganese, tin, zinc, lead, iron, magnesium, antimony, titanium with aromatic organic acids substituted by an alkyl group or an alkenyl group having a carbon number of 1-18, polybasic acids having a carbon number of 2-18 such as oxalic acid, adipic acid, malonic acid, palmitic acid, phthalic acid, isophthalic acid, trimellitic acid, and pyromellitic acid, or partial esters thereof; compounds of a dialkylated tin having a carbon number of 1-6 with saturated organic acids having a carbon number of 1-18, a compound of a dialkylated tin having a carbon number of 1-6 with unsaturated organic acids having a carbon number of 3-18; salts of saturated organic acids having a carbon number of 1-18 with aluminum, etc.; unsaturated organic acids or substituted aromatic organic acids having a carbon number of 3-18; polybasic acids having a carbon number of 2-18 such as oxalic acid, adipic acid, malonic acid, palmitic acid, phthalic acid, isophthalic acid, trimellitic acid, pyromellitic acid, or compounds with partial esters thereof; alkyl oxides having a carbon number of 1-18 of manganese, tin, zinc, lead, iron, magnesium, antimony, titanium, and aluminum, etc.

Of the above-described Lewis acids, the dialkyl tin compound is one of particularly useful compounds because those effectively accelerate the conversion of the isocyanate compound to polyurethanes by pulling out active hydrogens as shown in the following chemical formula.

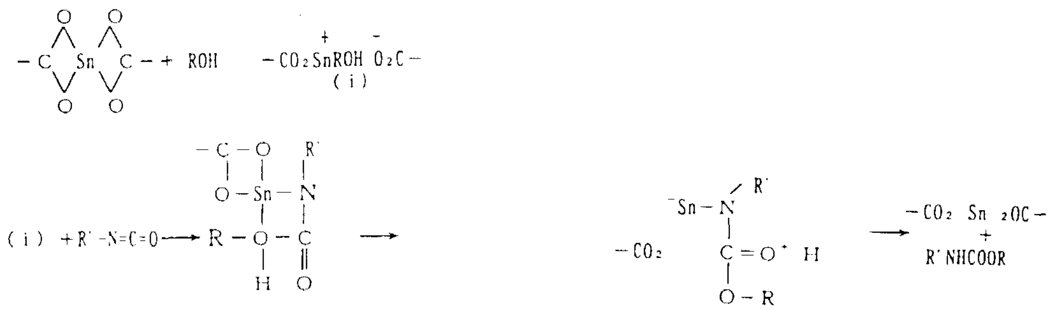

In the present invention, the use amount of the Lewis acids is $1x10^{-8}$-$1x10^{-2}$ equivalent, and preferably $1x10^{-6}$-$1x10^{-3}$ equivalent based on 1 equivalent of -NCO group in the cycloaliphatic polyisocyanate obtained without using phosgene through all the steps. Total amount of the acids containing the protonic acids and Lewis acids is preferably $1x10^{-8}$-$1x10^{-1}$ equivalent based on 1 equivalent of the NCO group.

Since the Lewis acids have a large catalytic effect, in the case that those are employed together with the protonic acids, there may be employed a small amount of the Lewis acids compared to the protonic acids.

In the case that the addition amount of the Lewis acids to be added is less than $1x10^{-8}$ based on 1 equivalent of the NCO group, there is only obtained a composition having a low effect for accelerating the conversion of a polyisocyanate into a polyurethane. On the other hand, in the case that the addition amount of the lewis acids to be added is more than $1x10^{-2}$ based on 1 equivalent of the NCO group, additional effects will be not only shown but also the protonic acids will react with a polyisocyanate to lower the quality and properties of the polyurethane.

In the present invention, as a method for the addition of the Lewis acids to the cycloaliphatic polyisocyanate, two methods are conducted as illustrated hereinafter.

A first method is carried out by previously adding a fixed amount of the Lewis acid to the cycloaliphatic polyisocyanate, whereby, there can be prepared a composition having a controlled reactivity.

A second method is carried out by feeding a Lewis acid and the cycloaliphatic polyisocyanate into a reactor together with other catalysts for the conversion of the cycloaliphatic polyisocyanate to a polyurethane when preparing, for example, a polyurethane coating, etc.

In the case, it is important that the water content of the Lewis acid to be mixed is previously lowered as small as possible.

If a large amount of water is mixed into the cycloaliphatic polyisocyanate, it will be converted into urea or an allophanate to bring out a lowering in the quality.

[(V) Polyurethane using the above-described cycloaliphatic polyisocyanate]

Hereinafter, there is illustrated the polyurethane using the above-described cycloaliphatic polyisocyanate as obtained hereinabove.

The cycloaliphatic polyisocyanate to be employed is the same as the cycloaliphatic polyisocyanate obtained in the above-described first aspect of the present invention.

The polyurethane is obtained by a reaction of the cycloaliphatic polyisocyanate with publicly-known polyols and/or polyamines.

As the polyols, there can be exemplified polyols such as diols, triols, tetraols, pentaols, and polyols having more functionalities from a viewpoint of a functionality.

As the polyols, there can be exemplified polyols having a low molecular weight, polyols having a high molecular weight, and polyester polyols having a high molecular weight from a viewpoint of a molecular weight.

As the polyamines, there can be exemplified polyamines such as diamines, triamines, tetramines, pentamines, and amines having more functionalities from a viewpoint of a functionality.

For the preparation of the polyurethanes, there can be further employed a variety of chain extenders, curing agents, crosslinking agents, water, etc. Further, catalysts are optionally employed.

As additives for the polyurethanes, there can be employed a variety of foaming agents, surface active agents, ultraviolet stabilizers, antioxidants, unti-electrostatic agents, and inorganic or organic fillers, etc.

The cycloaliphatic polyisocyanate in the present invention has a high reactivity compared to isophorone diisocyanate, etc., and those are advantageous from a viewpoint of reaction temperatures, the kind and a small use amount of catalysts.

Also, the polyurethanes obtained can be employed in a variety of conventional uses. Particularly, in cycloaliphatic structures by which a hard segment in a polyurethane is formed, a free mobility is high in the vicinity of

urethane bonds or urea bonds corresponding to the isocyanatemethyl group of 3-position in a starting raw polyisocyanate, whereby, a more flexible physical property is obtained against a change of temperatures, and those are appropriate for non-yellowing coatings and adhesives, etc.

[(VI) Adhesive composition comprising the above-described polyurethane]

Hereinafter, there is illustrated an adhesive composition comprising the polyurethane using the polyisocyanate as obtained hereinabove.
The kind of the cycloaliphatic polyisocyanate to be employed, if those are prepared by a method other than a direct preparation from phosgene and polyamines, is not particularly limited.
However, an effect is particularly large in the case of the cycloaliphatic polyisocyanate from which a non-yellowing polyurethane is obtained.
Accordingly, as the cycloaliphatic polyisocyanate to be employed in the adhesive composition comprising the polyurethane in the present invention, there are preferably employed the above-described cycloaliphatic polyisocyanate obtained without using phosgene. Those may be employed solely or in combination.
A polyurethane which can be employed for the adhesive composition of the present invention can be prepared by a polyaddition reaction of the above-described cycloaliphatic polyisocyanate with polyols such as polyether polyols, polyester polyols, and polycarbonatediols.
There are exemplified coating materials for optical fibers described in JP-A-01135872, JP-A-01172416, JP-A-01242613, JP-A-60195115, and JP-A-62007714 Official Gazettes; radiation ray-curable adhesives described in JP-A-60036577 (USP 509389) and JP-A-62116620 (USP 779838) Official Gazettes, etc.; cathode-electro deposition type adhesives described in JP-A-62074969, JP-A-620068861, JP-A-01161073, JP-A-61087765, JP-A-61145201, JP-A-62174277, JP-A-63043967, and JP-A-64085262 Official Gazettes; polyurethane adhesives described in JP-A-60250069, JP-A-61089274, JP-A-62164738, JP-A-02000619 (USP 549209), and JP-A-02004878 (DE 3803628) Official Gazettes, etc; single-liquid type baking urethane adhesives described in JP-A-54048777 (DE 2732622), JP-A-54048896 (DE 2732775), JP-A-56050969 (DE 2938855), JP-B-58004068 (DE 2346818), JP-A-59004658 (DE 3221558), and JP-B-01050265 (DE 3030544) Official Gazettes, etc.; dual-liquid type acryl urethane or polyester urethane adhesives which are obtained by using polyisocyanate compounds described in JP-A-58118553 (DE 3151853), JP-A-59095259, JP-A-61176625, JP-A-55143978 (DE 3151855), JP-A-58118575 (DE 3151855), JP-A-57078460 (DE 3033864), JP-A-60181114, JP-A-61028518, JP-B-61033852, JP-B-59002865, JP-A-47005837 (DE 2043493), JP-A-50041929 (DE 2325824), JP-A-60255861, JP-A-61283669, JP-A-62030160, and JP-A-62277472 Official Gazettes, etc.; urethane powder adhesives which are obtained by using block isocyanate curing agents described in JP-A-53121773 (DE 2712931), JP-A-54084584 (DE 2751805), JP-A-54044670 (DE 2729704), JP-B-85041062 (DE 2929224), JP-B-89005627 (DE 3030558), JP-A-56049761 (DE 2929150), JP-A-60047079 (DE 3322718), JP-A-60053511 (DE 3328131), JP-A-57063324 (DE 3030539), JP-B-81051190 (DE 2105777), and JP-B-86031744 (DE 2735497) Official Gazettes, etc.; primer adhesives and anti-staining adhesives using urethane prepolymers described in JP-A-62146965 (US 808762/A), JP-A-60013858 (DE 3322723), JP-A-62169864, and JP-A-62246972 Official Gazettes, etc.
Of the urethane adhesives disclosed in the prior arts, a method for the preparation of the polyurethane resin composition is illustrated in more detail. In the methods, chain extenders may be optionally employed together with the above-described mixture.
As the chain extenders, there are low molecular weight compounds having active hydrogens. As the specific examples, there are exemplified ethylene glycol, propylene glycol, 1,4-butylene glycol, 2-methylpropanediol, neopentyl glycol, pentanediol, 1,6-hexanediol, ethylenediamine, propylenediamine, hydrazine, isophorone diamine, metaphenylene diamine, 4,4'-diaminodiphenyl methane, diaminodiphenyl sulfone, 3,3'-dichloro-4,4'-diaminodiphenyl methane, 3-aminomethylcyclopentyl amine, 3-aminomethylcyclohexyl amine, 3-aminomethyl-5-methyl cyclohexyl amine, 3-aminomethylcycloheptyl amine, 3-aminomethyl-5,5-dimethylcyclohexyl amine, 3-aminomethylcyclooctyl amine, 3-aminomethyl-5-methylcyclooctyl amine, and 5-phenyl-3-aminomethyl cyclohexylamine, etc.
As the method for the preparation of the polyurethane in the present invention, there can be applied a prepolymer method in which a variety of the above-described polyols are allowed to react with an excessive amount of the above-described cycloaliphatic polyisocyanate to prepare a prepolymer having terminal isocyanate groups, followed by allowing to react with the chain extenders such as diols or diamines, and a one-shot method in which all components are simultaneously mixed to prepare a polyurethane.
The method can be carried out in the absence or presence of solvents. Inert solvents are preferably employed.
Specifically, there are toluene, ethyl acetate, butyl acetate, methylethyl ketone, dimethylformamide, and tetrahydrofran, etc.
Further, in the conversion into a polyurethane, catalysts can be also employed. For example, there are exemplified organic tin compounds such as tin octylate, dibutyl tindilaurate, or tertiary amines such as N-methylmorpholin,

and triethylamine.

Thus-prepared polyurethane is particularly excellent in an anti-yellowing property.

The adhesives composition comprising the above-described polyurethane resin composition in the present invention is improved in physical properties such as weatherability, corrosion resistance, and heat resistance.

The above-described cycloaliphatic polyisocyanate obtained from the aliphatic polyamines as described hereinabove can be applied also to a bifunctional, trifunctional, and more functional isocyanate compounds.

[Examples]

Hereinafter, although the present invention is specifically illustrated by Examples, the present invention is not limited thereto.

Commercially supplied polyols employed in the Examples are as follows.

(a) Polycaprolactone diol having a number average molecular weight of 2,000 (a trade name, PCL-220 manufactured by Daicel Chemical Industries, Ltd.)
(b) Polycaprolactone triol having a number average molecular weight of 540 (a trade name, Placcel 305 manufactured by Daicel Chemical Industries, Ltd.)
(c) Polytetramethylene glycol having a number average molecular weight of 2,000 (a trade name, PTG 2000 manufactured by Hodogaya Kagaku, Ltd.)
(d) Polytetramethylene glycol having a number average molecular weight of 1,000 (a trade name, PTG 1000 manufactured by Hodogaya Kagaku, Ltd.)
(e) Copolymer of tetrahydrofran-propyleneoxide having a number average molecular weight of 2,000 (a trade name, PPTG 2000 manufactured by Hodogaya Kagaku, Ltd.)
(f) Polypropylene glycol having a number average molecular weight of 400 (a trade name, Sannix PP-400 manufactured by San-yo Kasei, Ltd.)

[Examples for the first aspect of the present invention]

<Preparation Example I-1/Preparation of a catalyst for oxidation>

A solution composed of 45.6 g of 85%-phosphoric acid and 60 ml of water was mixed with a solution in which 43.90 g of sodium metavanadinate and 49.32 g of sodium molybdic acid were dissolved in 300 ml of water by heating to 95°C, followed by being maintained at 95°C while agitating for 1 hour. After cooled to 0°C, a solution of 35.6 g of ammonium chloride and 126 ml of water were added to obtain a brown-colored precipitate. The precipitate was filtered, and it was further dissolved twice in water to recrystallize to obtain an ammonium salt of a heteropolyacid. The ammonium salt of a heteropolyacid obtained was analyzed to identify the composition of $(NH_4)_5H_6[PV_8Mo_4O_{40}]$-9.6$H_2O$.

1800 mg of an activated carbon was added to a solution in which 200 mg of the ammonium salt of a heteropolyacid is dissolved in 4,000 ml of water, followed by placing at room temperatures while agitating for 1 hour. After filtered and washed with 4,000 ml of water, a catalyst carried on an activated carbon was obtained by drying at 80°C.

<Synthesis Example I-1/Synthesis of 3-formyl-5,5-dimethyl cyclohexenone>

A glass-made flask (capacity of 5 liter) was charged with 175 g of the catalyst prepared hereinabove, 138 g of isophorone, and 2,000 g of toluene, followed by allowing to react for 20 hours while refluxing under an oxygen atmosphere. As a result of an analysis by a gas chromatographic method, 93% of isophorone reacted, and 62% of unreacted isophorone converted into 3-formyl-5,5-dimethyl cyclohexenone (yield of 58% by weight).

<Synthesis Example I-2/synthesis of 3-aminomethyl-5,5-dimethyl cyclohexylamine MDMCHDA>

An electromagnetically-agitating type autoclave (capacity of 3 liter) was charged with a solution in which 50 g of 3-formyl-5,5-dimethylcyclohexenone is dissolved in 1,000 g of methanol, 10 g of a Raney nickel catalyst, and 150 g of ammonia. After temperature was elevated to 120°C, a reaction was carried out at hydrogen partial pressure of 50 $kgf/cm_2$ and rotating speed of 800-1,000 rpm in an agitator for 2 hours.

After the completion of the reaction, the autoclave was cooled and pressure was discharged, and a reaction liquid was taken out, followed by filtering the catalyst and distilling off methanol at ordinary pressure to obtain 41 g of a substance.

The substance obtained contained 97.4% by weight of 3-aminomethyl-5,5-dimethylcyclohexylamine (MDMCHDA). The substance was analyzed by a mass spectrometry, an elementary analysis, and an infrared spectrum

absorption analysis.

Results of a mass spectrometry analysis:

Molecule ion peaks (m/e) in the mass spectra were observed at 156 (theoretical molecular weight of 156), 127, 126, 113, 70, 56, and 43.

Results of an elementary analysis:

Results are C=69.5, N=12.7, H=17.8% by weight, respectively, and elementary composition is $C_9N_2H_{20}$.

<Production Example I-1/Production of 3-methoxycarbonyl aminomethyl-5,5-dimethyl-1-methoxycarbonylaminocyclohexane MDMCHDC>

A round bottom flask equipped with an agitator was charged with 78 g of MDMCHDA obtained by the above-described method and 360 g of dimethylcarbonate (DMC), followed by elevating temperature to 70°C while agitating under a nitrogen atmosphere.

Subsequently, 8.9 g of methanol solution containing 28% by weight of sodium methylate was added dropwise, and aging was further continued for 50 minutes to obtain a reaction crude liquid.

As a result of an analysis by a gas chromatography, it was identified that MDMCHDC of the following formula (8) corresponding to MDMCHDA is produced in a yield of 92% by weight based on the MDMCHDA which is a starting raw material.

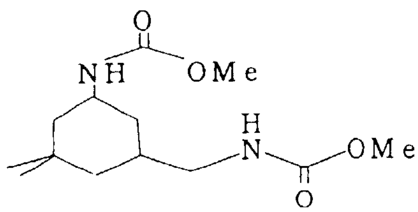

(8)

<Example I-1/Production of 3-isocyanatemethyl-5,5-dimethyl cyclohexylisocyanate (MDMCHDI)/Production of MDMCHDI by a method without using phosgene>

300 ml of a flask was set up under a glass-made Oldarshaw column having 10 stages equipped with a reflux condenser, and 500 g of a partially-hydrogenated triphenyl (a trade name, Therm S900 manufactured by Shin-Nittetsu Kagaku, Ltd.) and 0.05 g of manganese acetate $4H_2O$ were fed, followed by heating under a reduced pressure and adjusting the degree of reduced pressure so that a liquid boils at 230°C.

Subsequently, the MDMCHDC obtained as described above was added dropwise from a dual-tube type dropping funnel heated by a heating medium into the flask at a temperature where MDMCHDC maintains fluidity in dropping rate of 60 g/hour. After initiation of dropping, a MDMCHDI (represented by Formula (9))-rich liquid elevated to the column, and the MDMCHDI was distilled out of a top of the column at a distilling rate adjusted to dropping rate of the MDMCHDC.

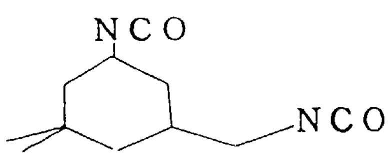

(9)

After being operated for 2 hours, distillation was continued until the MDMCHDI becomes not produced, followed by terminating operation. Distillate was collected, and analyzed by gas chromatography. As a result, it was identified that yield of the MDMCHDI was 93% by weight based on the starting MDMCHDA.

Further, it was identified that the distillate is MDMCHDI by an elementary analysis, an infrared absorption spectrum analysis, an NMR spectra, and the content of isocyanate group described below.

Result of the elementary analysis: C=64.0, H=7.7, N=13.0, and assuming that the residue is O, it is 15.3. Composition ratio of elements is $C_{11}H_{16}N_2O_2$.

The content of isocyanate group in the cycloaliphatic polyisocyanate measured by a titration method was 39.4% by weight compared to a theoretical value of 40.4% by weight.

<Example I-2/Production of MDMCHDI by a phosgene method>

78 g of the MDMCHDA obtained by the Synthesis Example I-2 was dissolved in 500 g of orthodichlorobenzene, and 1 mol of phosgene was blown at 0°C over 1 hour, followed by carrying out a reaction for removing hydrogen chloride at 40°C and reduced pressure and further carrying out a reaction for removing hydrogen chloride at 130°C to obtain a reaction product. The reaction product was filtered to obtain a crystal, and then recrystallized.
Content of the MDMCHDI in a refined crystal obtained was 98% based on area as a result of a gas chromatographic analysis.

[Examples for the second aspect of the present invention]

<Synthesis Example II-1/Synthesis of dimethyl carbonate by a method without phosgene>

A reaction for the preparation of dimethyl carbonate by a method without phosgene was carried out using a Teflon-coated autoclave having an internal capacity of 5 liter.
A mixed gas consisting of 47.5% by volume of nitrogen, 30% by volume of carbon monoxide, and 22.5% by weight of argon/oxygen (oxygen concentration of 33% by volume) were fed into the autoclave at 12.0 $kg/cm^2$ and allowed to react by the use of 526 ml of a methanol solution containing palladium chloride in an amount of 7.5 mmol/l, cuprous acetate in an amount of 187.5 mmol/l and magnesium chloride in an amount of 187.5 mmol/l as a catalyst at 130°C for 1 hour.
Crude reaction liquid was distilled to obtain dimethyl carbonate. The same procedures were repeated 20 times, whereby, 234 g of dimethyl carbonate was obtained.
It was identified that the content of chlorine in the dimethyl carbonate obtained is approximately 11 ppm by a measurement using an ion chromatograph (IC-500).

<Synthesis Example II-2/Synthesis of dimethyl carbonate by a method without phosgene>

The reaction in the Synthesis Example II-1 was followed 40 times except that sodium chloride was employed in place of magnesium chloride to obtain approximately 720 g of dimethyl carbonate. In the case, the reaction proceeded in a state that catalyst suspends in a liquid. Chlorine content in dimethyl carbonate obtained was approximately 8 ppm.

<Production Example II-1/Production No. 1 of 3-methoxycarbonyl aminomethyl-5,5-dimethyl-1-methoxycarbonylaminocyclohexane (MDMCHDC)>

A round bottom flask equipped with an agitator was charged with 78 g of 3-aminomethyl-5,5-dimethylcyclohexylamine (MDMCHDA) obtained in the Synthesis Example I-2 and 360 g of dimethyl carbonate obtained in the Synthesis Example II-2, followed by elevating temperature to 70°C while agitating and streaming nitrogen.
Subsequently, 8.9 g of methanol solution containing 28% by weight of sodium methylate was added dropwise for 10 minutes, and aging was further continued for 50 minutes to obtain a reaction crude liquid. As a result of an analysis by a gas chromatography, it was identified that MDMCHDC is produced in a yield of approximately 98% based on MDMCHDA.
Evaporation was carried out in order to remove low boiling components in the above-described reaction crude liquid, and further, followed by washing by water to obtain a refined MDMCHDC which is employed as a starting material in Example II-1.

<Production Example II-2/Production No. 2 of 3-methoxycarbonyl aminomethyl-5,5-dimethyl-1-methoxycarbonylaminocyclohexane (MDMCHDC)>

A round bottom flask equipped with an agitator was charged with 360 g of dimethyl carbonate obtained in the Synthesis Example II-2, followed by elevating temperature to 70°C while agitating and streaming nitrogen.
Subsequently, 8.9 g of 28%-methanol solution of sodium methylate and 78 g of MDMCHDA were fed in an equal feeding rate by 2 sets of pumps over 2 hours. During feeding, a temperature of reaction crude liquid was maintained at 70°C.
After the completion of feeding, aging was carried out for 3 hours at the same temperature. Subsequently, the reaction crude liquid neutralized by 85%-phosphoric acid was analyzed by a gas chromatography to identify that MDMCHDC corresponding to MDMCHDA is produced in a yield of approximately 99% based on MDMCHDA, and in a yield of approximately 99% based on DMC consumed.

<Production Example II-3/Production No. 3 of 3-methoxycarbonyl aminomethyl-5,5-dimethyl-1-methoxycarbonylaminocyclohexane (MDMCHDC)>

Following reaction was carried out using the same apparatus as in the Production Examples II-1 and II-2.
Into a reaction vessel maintained at 70°C in which 723.1 g of DMC is fed, 163.1 g of MDMCHDA obtained in the Synthesis Example I-2 and 17.3 g of 28%-methanol solution of sodium methylate were fed.
The 28%-methanol solution was added dropwise in an interval of 30 minutes after divided into 4 portions.
After the dropwise addition, aging was further carried out for 1 hour at 70°C. As a result of titration by 1/10 normal of $HClO_4$ and a compensation of methanol component, it was identified that MDMCHDA is converted in a conversion of 99.6%
After that, a reaction crude liquid was analyzed by a gas chromatography after neutralized by 7.5 g of 85%-phosphoric acid to identify production of MDMCHDC in a yield of 98.8% based on MDMCHDA.

<Example II-1/Production No. 1 of MDMCHDI by a method without using phosgene>

300 ml of a flask was set up under a glass-made Oldarshaw column having 10 stages equipped with a reflux condenser, and 100 g of a partially-hydrogenated triphenyl (a trade name, Therm S900 manufactured by Shin-Nittetsu Kagaku, Ltd.) and 0.05 g of manganese acetate-4$H_2O$ were fed, followed by heating under a reduced pressure and adjusting the degree of reduced pressure so that a liquid in the flask boils at 230°C.
Subsequently, MDMCHDC obtained in the Production Example II-1 was added dropwise from a dual-tube type dropping funnel heated by a heating medium into the flask at a temperature where MDMCHDC maintains fluidity in a dropping rate of 66.8 g/hour.
After initiation of dropping, an MDMCHDI-rich liquid elevated to the column, and MDMCHDI was distilled out of a top of the column at a distilling rate adjusted to dropping rate of MDMCHDC.
After being operated for 2 hours, distillation was continued until MDMCHDI becomes not produced, followed by terminating an operation.
Distillate was collected, and it was analyzed by a gas chromatography. As a result, it was identified that yield of MDMCHDI was 76% based on starting MDMCHDC.
Further, it was identified that the distillate is MDMCHDI by an elementary analysis, an infrared absorption spectrum analysis, NMR spectra, and the content of isocyanate group described below.
Result of elementary analysis: C=64.0, H=7.7, N=13.0, and assuming that the residue is O, it is 15.3. Composition ratio of elements is $C_{11}H_{16}N_2O_2$.
It is to be noted that the content of isocyanate group in the MDMCHDI measured by a titration method was 39.3% by weight compared to a theoretical value of 40.4% by weight.

<Example II-2/Production No. 2 of MDMCHDI by a method without using phosgene>

200 ml of a glass-made reboiler was set up under a glass-made Oldarshaw column having 20 stages equipped with a reflux condenser to carry out a continuous thermal decomposition of MDMCHDC obtained in the Production Example II-1. m-terphenyl was employed as a solvent.
200 ml of m-terphenyl and 10 ppm of anhydrous manganese acetate based on m-terphenyl were fed in the reboiler, followed by heating until becoming an evaporation state under a reduced pressure of 10 torr.
Subsequently, a mixture of 59.0% by weight of MDMCHDC with 41.0% by weight of m-terphenyl was fed in a feeding rate of 113.8 g/hour. The m-terphenyl solution of MDMCHDC was fed at fifth stage from bottom of the Oldarshaw column.
MDMCHDI which is a product was discharged from a top of distillation column, and a operation was carried out at a discharging rate from bottom where a liquid surface in the reboiler is maintained at a constant level.
Through the condenser, warm water of 60°C was flown, and methanol was distilled off as a gas while condensing MDMCHDI.
Through the reaction, a methanol solution of 125 ppm of anhydrous manganese acetate was fed while adjusting the amount of a liquid discharged from the bottom so that the concentration of anhydrous manganese acetate in the reboiler is maintained at 10 ppm.
The methanol solution of 125 ppm of anhydrous manganese acetate was prepared by dissolving anhydrous manganese acetate into methanol in 1% by weight, and then diluting 80 times by MDMCHDI.
The liquid was homogeneous and a low viscous liquid.
The methanol solution of anhydrous manganese acetate was fed at 13th stage from bottom of the Oldarshaw column.
At a period when temperature in the column, the amount of a distillate and liquid discharged from bottom,

and composition became stable, 52 g of a distillate was distilled off for 1 hour.

The composition of the distillate was 98.2% by weight of MDMCHDI and 0.05% by weight of m-terphenyl.

[Measurement of total chlorine content]

Total chlorine content in MDMCHDI obtained was determined based on a method regulated in ASTM D-1638. Total chlorine content in MDMCHDI obtained in the Example II-1 was 0.2 ppm, and total chlorine content in MDMCHDI obtained in the Example II-2 was also 0.2 ppm. On the other hand, it was identified that the total chlorine content in MDMCHDI obtained by a phosgene method is 215 ppm as a result measured by the same method.

[Examples for the third aspect of the present invention]

<Synthesis Example III-1/Synthesis of MDMCHDC from MDMCHDA and DMC prepared without using phosgene through all steps>

211 g of DMC prepared in the Synthesis Example II-1 was fed into a round bottom flask equipped with an agitator, followed by elevating a temperature to 70°C while agitating under nitrogen streaming.

Subsequently, 5.22 g of 28%-methanol solution of sodium methylate and 45.7 g of MDMCHDA prepared in the Synthesis Example 1-2 were fed by 2 sets of pumps over 70 minutes in a equal feeding rate. During feeding, a temperature in reaction was maintained at 70°C. Further, after the completion of feeding, aging was carried out for 3 hours at the same temperature. Subsequently, a reaction crude liquid neutralized by phosphoric acid was analyzed by a gas chromatography to identify that MDMCHDC is produced in a yield of approximately 99% based on MDMCHDA, and in a yield of approximately 99% based on DMC consumed.

Evaporation was carried out in order to remove low boiling components in the above-described reaction crude liquid, followed by washing by water to obtain a refined MDMCHDC which is employed as a raw material in Production Example III-1.

<Synthesis Example III-2>

The same synthesis was followed as in the Synthesis Example III-1 except that the DMC prepared in the Synthesis Example II-2 was employed in place of the DMC prepared in the Synthesis Example II-1 to obtain MDMCHDC in the approximately same yield.

Evaporation was carried out in order to remove low boiling components in the above-described reaction crude liquid, followed by washing by water to obtain a refined MDMCHDC which is employed as a raw material in Production Example III-2.

<Production Example III-1/Production of MDMCHDI by a thermal decomposition of MDMCHDC prepared from MDMCHDA and DMC prepared by a method without using phosgene through all steps>

200 ml of a glass-made reboiler was set up under a glass-made Oldarshaw column having 10 stages equipped with a reflux condenser to carry out a continuous thermal decomposition of MDMCHDC obtained in the Synthesis Example III-1. m-terphenyl was employed as a solvent.

117 ml of m-terphenyl and 10 ppm g of anhydrous manganese acetate based on m-terphenyl were fed in the reboiler, followed by heating until becoming an evaporation state under a reduced pressure of 10 torr.

Subsequently, a mixture of 59.0% by weight of MDMCHDC with 41.0% by weight of m-terphenyl was fed in a feeding rate of 120 g/hour.

MDMCHDI which is a product was discharged from a top of distillation column, and a operation was carried out at a discharging rate from bottom where a liquid surface in the reboiler is maintained at a constant level.

Through the condenser, warm water of 60°C was flown, and methanol was distilled off as a gas while condensing MDMCHDI.

Through the reaction, a methanol solution of 125 ppm of anhydrous manganese acetate was fed while adjusting the amount of a liquid discharged from the bottom so that the concentration of anhydrous manganese acetate in the reboiler is maintained at 10 ppm.

At a period when temperature in the column, the amount of a distillate and liquid discharged from bottom, and composition became stable, 17.0 g of a distillate was distilled off for 1 hour.

The composition of the distillate was 77.2% by weight of diisocyanates, 22.7% by weight of a corresponding monoisocyanate, and 0.05% by weight of m-terphenyl.

Temperature of reaction vessel was 245°C.

Conversion of MDMCHDC was 99% by weight, and conversion of high boilers from MDMCHDC was 6% by weight.

Yield of MDMCHDI and a monoisocyanate was 74% and 19% by weight based on MDMCHDC fed, respectively.

Chlorine content in the MDMCHDI obtained was 0.2 ppm.

<Production Example III-2>

A solution was prepared by dissolving anhydrous manganese acetate into methanol in 1% by weight. The methanol solution of 125 ppm of anhydrous manganese acetate was prepared by dissolving anhydrous manganese acetate into methanol in 1% by weight, and then diluting 80 times by MDMCHDI. The liquid was homogeneous and a low viscous liquid.

The same operations were followed as in Production Example III-1 except that MDMCHDC was fed at 5th stage from bottom of distillation column using the catalyst solution, and the catalyst solution was fed at 13th stage from bottom of distillation column.

At a period when temperature in the column, the amount of a distillate and liquid discharged from bottom, and composition became stable, a distillate was distilled off for 1 hour. The composition of the distillate was 98.1% by weight of MDMCHDI, 1.8% by weight of a monoisocyanate (MDMCHMI), and 0.05% by weight of m-terphenyl.

Conversion of MDMCHDC was 99% by weight, and conversion of high boilers from MDMCHDC was 2% by weight.

Yield of MDMCHDI and the monoisocyanate (MDMCHMI) was 95.5% and 11.5% by weight based on MDMCHDC fed, respectively.

The distillate MDMCHDI obtained was further refined until the purity of 99.7% by weight by a batchwise distillation to employ as a raw material for Examples described hereinafter.

Chlorine content in the MDMCHDI obtained was 0.1 ppm.

Further, it was identified that the distillate is MDMCHDI by an elementary analysis, an infrared absorption spectrum analysis, NMR spectra, and the content of isocyanate group described below.

Result of elementary analysis: C=64.0, H=7.7, N=13.0, and assuming that the residue is O, it is 15.3. Composition ratio of elements is $C_{11}H_{16}N_2O_2$.

It is to be noted that the content of isocyanate group in the cycloaliphatic polyisocyanate measured by a titration method was 39.3% by weight compared to a theoretical value of 40.4% by weight.

<Example III-1>

$5x10^{-4}$ equivalent of acetic acid was added based on isocyanate group in 100 g of MDMCHDI obtained in Production Example III-2, followed by dissolving in o-xylene to prepare an MDMCHDI solution of 2.0 mol/liter.

Separately, a special reagent grade n-butanol was dissolved in o-xylene to prepare a n-butanol solution of 2.0 mol/liter.

A 50 ml-Pyrex glass-made reaction vessel equipped with a condenser and a mouth for taking out a sample was charged with 5 ml of the above-described MDMCHDI solution and 10 ml of n-butanol solution by precisely measuring with a hole pipet, followed by precisely adding 5 ml of diphenylether/o-xylene solution having the concentration of 0.8 mol/liter with a hole pipet, which was in advance prepared.

While sufficiently agitating and heating a reaction solution in an oil bath controlled at temperature of 60± 1°C, MDMCHDI was allowed to react with n-butanol to proceed the reaction to a corresponding diurethane compound.

Residual concentration of MDMCHDI and n-butanol in the reaction solution was monitored by a gas chromatographic analysis with a lapse of time to measure the reaction to a corresponding diurethane compound.

As a result, reactivity of MDMCHDI was measured by measuring the conversion from isocyanate groups to urethane bonds at a period of 3 hours after the reaction. Results in the measurement are shown in Table III-1.

<Examples III-2 to III-6>

The same procedures were repeated as in the Example III-1 except that protonic acids shown in Table III-1 were added to MDMCHDI obtained in Production Example III-2 in a determined amount to measure a reactivity in conversion into a polyurethane. Results of the measurements are shown in Table III-1.

<Comparative Example III-1>

The same procedures were repeated as in the Example III-1 except that acetic acid was not added to MDMCHDI obtained by a DMC method in Production Example III-2 to measure a reactivity in conversion into a polyurethane. Results of the measurements are shown in Table III-1.

<Comparative Example III-2>

The same procedures were repeated as in the Example III-1 except that MDMCHDI prepared by a phosgene method was employed, and acetic acid was not employed to measure a reactivity in conversion into a polyurethane. Results of the measurements are shown in Table III-1.

Table III-1

| | EXAMPLE III-1 | EXAMPLE III-2 | EXAMPLE III-3 | EXAMPLE III-4 | EXAMPLE III-5 |
|---|---|---|---|---|---|
| Diisocyanate | D-MDMCHDI | D-MDMCHDI | D-MDMCHDI | D-MDMCHDI | D-MDMCHDI |
| Kind of protonic acids | AA | PA | ADA | DBS | PHA |
| Equivalent of protonic acids added (equivalent/$10^{-4}$ equivalent of NCO group) | 5.0 | 5.5 | 5.4 | 2.7 | 1.7 |
| Mixing ratio of NCO/OH in the conversion into polyurethane | 1.0 | 1.0 | 1.0 | 1.0 | 1.0 |
| Reaction temperature (°C) | 60 | 60 | 60 | 60 | 60 |
| Reaction proportion of MDMCHDI after 3 hours | 2.18 | 2.16 | 2.16 | 2.2 | 1.56 |

Table III-1 (continued)

| | EXAMPLE III-6 | EXAMPLE III-7 | EXAMPLE III-8 | COMPARATIVE EXAMPLE III-4 | COMPARATIVE EXAMPLE III-5 |
|---|---|---|---|---|---|
| Diisocyanate | D-MDMCHDI | D-MDMCHDI | D-MDMCHDI | D-MDMCHDI | P-MDMCHDI |
| Kind of protonic acids | DPA | AA | AA | -- | -- |
| Equivalent of protonic acids added (equivalent/$10^{-4}$ equivalent of NCO group) | 5.0 | 0.05 | 52.0 | -- | -- |
| Mixing ratio of NCO/OH in the conversion into polyurethane | 1.0 | 1.0 | 1.0 | 1.0 | 1.0 |
| Reaction temperature (°C) | 60 | 120 | 70 | 60 | 60 |
| Reaction proportion of MDMCHDI after 3 hours | 1.86 | 1.69 | >2.2 | 1 | 1.93 |

In the Table III-1 and Table III-1 (continued), abbreviations are as follows.

D-MDMCHDI: 3-isocyanatemethyl-5,5-dimethylcyclohexyl isocyanate prepared by a dimethyl carbonate method
P-MDMCHDI: 3-isocyanatemethyl-5,5-dimethylcyclohexyl isocyanate prepared by a phosgene method
AA: acetic acid

PA: propionic acid
ADA: adipic acid
DBS: dodecylbenzene sulphonate
PHA: phosphoric acid
DPA: dimethyl phosphinic acid

It is to be noted that reaction proportion of MDMCHDI after 3 hours is the reaction proportion based on 1 of the reaction proportion of MDMCHDI in the Comparative Example III-1.

<Example III-7>

A reaction apparatus equipped with a condenser, an agitator, thermometer, and a tube for supplying nitrogen gas was charged with 104.1 g of MDMCHDI obtained in Production Example III-2, $5.4 \times 10^{-4}$ equivalent of acetic acid based on isocyanate group, and 1,000 g of a polytetramethyleneglycol having a number average molecular weight of 2,000 (PTG 2000 manufactured by Nihon Polyurethane Kogyo Co., Ltd.), followed by being immediately heated and allowed to react for conversion into a polyurethane at 120°C.
Residual amount of MDMCHDI in a reactant was monitored with a lapse of time by a gas chromatographic analysis method to measure the conversion into a polyurethane. As a result, even though the reaction was continued for 3 hours after attained to a reaction temperature of 120°C, a large decrease was not observed in the concentration of the residual MDMCHDI and the concentration of isocyanate group.
Results of the measurements are shown in Table III-1.

<Example III-8>

The same conditions in the conversion into a polyurethane were followed as in the Example III-7, except that a reaction temperature was changed to 70°C.
Residual amount of MDMCHDI in a reactant was monitored with a lapse of time by a gas chromatographic analysis method to measure the conversion into a polyurethane. As a result, even though the reaction was continued for 9 hours after attained to a reaction temperature of 70°C, a large decrease was not observed in the concentration of the residual MDMCHDI and the concentration of isocyanate group.
Results in the measurements are shown in Table III-1.
It is to be noted that Reaction proportion of MDMCHDI after 3 hours is the reaction proportion based on 1 of reaction proportion of MDMCHDI in the Comparative Example III-1.

<Example III-9>

The same conditions in the conversion into a polyurethane were followed as in the Example III-7, except that a polycaprolactone diol having a number average molecular weight of 2,000 (Placcel 220 manufactured by Daicel Chemical Industries, Ltd.) was employed in place of the polytetramethyleneglycol.
Residual amount of MDMCHDI in a reactant was monitored with a lapse of time by a gas chromatographic analysis method to measure the conversion into a polyurethane. As a result that the reaction was continued, there was obtained a urethane polymer having the concentration of the residual MDMCHDI of 2.4% by weight and the concentration of isocyanate group of 3.4% by weight at a period of 2 hours after attained to a reaction temperature of 120°C.
After that, although the reaction was continued, a large decrease was not observed in the concentration of the residual MDMCHDI and the concentration of isocyanate group.

<Example III-10>

A reaction apparatus equipped with a condenser, an agitator, a dropping funnel, thermometer, and a tube for supplying nitrogen gas was charged with 2,000.0 g of MDMCHDI obtained in Production Example III-2, $5.4 \times 10^{-4}$ equivalent of acetic acid based on isocyanate group, followed by heating to 50°C. 190.8 g of trimethylolpropane (manufactured by Mitsubishi Gas Kagaku Kogyo, Ltd.) thermally melted at 70°C was added dropwise over 3 hours, and reaction temperature elevated to 70°C.
After the completion of dropwise addition, the concentration of isocyanate group in a reactant was monitored with a lapse of time by a titration analysis method to measure the conversion into a polyurethane.
At a period of 3 hours after the completion of dropwise addition, there was obtained a mixture of a urethane polymer/monomer having the concentration of isocyanate group of 29.5% by weight. After that, although the reaction was continued, a large decrease was not observed in the concentration of isocyanate group.

<Comparative Example III-3>

A reaction apparatus equipped with a condenser, an agitator, thermometer, and a tube for supplying nitrogen gas was charged with 104.1 g of MDMCHDI obtained in Production Example III-2 and 1,000 g of a polytetramethyleneglycol having a number average molecular weight of 2,000 (PTG 2000 manufactured by Nihon Polyurethane Kogyo Co., Ltd.), followed by being immediately heated and allowed to react for conversion into a polyurethane at 120°C.

As a result, even though the reaction was continued for 6 hours after attained to a reaction temperature of 120°C, a large decrease was not observed in the concentration of the residual MDMCHDI and the concentration of isocyanate group.

<Comparative Example III-4>

The same conditions in the conversion into a polyurethane were followed as in the Comparative Example III-3, except that a reaction temperature was changed to 70°C.

Residual amount of MDMCHDI in a reactant was monitored with a lapse of time by a gas chromatographic analysis method to measure the conversion into a polyurethane. As a result, there was obtained a urethane polymer having the concentration of the residual MDMCHDI of 7.7% by weight and the concentration of isocyanate group of 4.1% by weight at a period of 12 hours after attained to a reaction temperature of 70°C, which was not a desired urethane prepolymer.

<Comparative Example III-5>

The same conditions in the conversion into a polyurethane were followed as in the Comparative Example III-4, except that a polycaprolactone diol having a number average molecular weight of 2,000 (Placcel 220 manufactured by Daicel Chemical Industries, Ltd.) was employed in place of the polytetramethyleneglycol.

Residual amount of MDMCHDI in a reactant was monitored with a lapse of time by a gas chromatographic analysis method to measure the conversion into a polyurethane. Although the reaction was continued, a large decrease was not observed in the concentration of the residual MDMCHDI and the concentration of isocyanate group at a period of 4 hours after attained to a reaction temperature of 120°C.

<Comparative Example III-6>

A reaction apparatus equipped with a condenser, an agitator, a dropping funnel, a thermometer, and a tube for supplying nitrogen gas was charged with 2,000 g of MDMCHDI obtained in Production Example III-2, followed by heating at 50°C. 190.8 g of trimethylolpropane (manufactured by Mitsubishi Gas Kagaku Kogyo, Ltd.) thermally melted at 70°C was added dropwise over 3 hours, and reaction temperature elevated to 70°C.

After the completion of dropwise addition, the concentration of isocyanate group in a reactant was monitored with a lapse of time by a titration analysis method to measure the conversion into a polyurethane. At a period of 9 hours after the completion of dropwise addition, although there was obtained a mixture of a urethane prepolymer/monomer having the concentration of isocyanate group of 31.4% by weight, viscosity is very high in the mixture of a urethane polymer/monomer which has a large molecular weight distribution compared to a desired mixture of a urethane prepolymer/monomer.

<Comparative Example III-7>

The same conditions in the conversion into a polyurethane were followed as in the Comparative Example III-4, except that MDMCHDI by a phosgene method obtained in Example I-2 was employed. At a period of 4 hours after attained to a reaction temperature of 120°C, there was obtained a urethane polymer having the concentration of the residual MDMCHDI of 4.0% by weight and the concentration of isocyanate group of 3.5% by weight, which is desired.

After that, although the reaction was continued, a large decrease was not observed in the concentration of the residual MDMCHDI and the concentration of isocyanate group.

[Examples for the fourth aspect of the present invention]

<Example IV-1>

As a Lewis acid, $1.12 \times 10^{-4}$ equivalent of dibutyltin dilaurate based on isocyanate group was added to 100 g of the above-described MDMCHDI obtained in Production Example III-2, followed by dissolving in o-xylene to prepare

a solution of MDMCHDI having the concentration of 2.0 mol/liter.

Separately, a special reagent grade n-butanol was dissolved in o-xylene to prepare n-butanol solution of 2.0 mol/liter.

A 50 ml-Pyrex glass-made reaction vessel equipped with a condenser and a mouth for taking out a sample was charged with 5 ml of the above-described MDMCHDI solution and 10 ml of n-butanol solution by precisely measuring with a hole pipet, followed by precisely adding 5 ml of diphenylether/o-xylene solution having the concentration of 0.8 mol/liter with a hole pipet, which was in advance prepared.

While sufficiently agitating and heating a reaction solution in an oil bath controlled at temperature of $60 \pm 1°C$, MDMCHDI was allowed to react with n-butanol to proceed the reaction for the conversion into a polyurethane.

Residual concentration of MDMCHDI and n-butanol in the reaction solution was monitored by a gas chromatographic analysis method with a lapse of time to measure the reaction for the conversion into a polyurethane.

As a result, reactivity of MDMCHDI was measured by measuring the conversion from isocyanate groups to urethane bonds at a period of 3 hours after the reaction. Results in the measurement are shown in Table IV-1.

<Examples IV-2 to IV-6>

The same procedures were repeated as in the Example IV-1 except that Lewis acids shown in Table IV-1 were added to MDMCHDI by a DMC method obtained in the above-described Production Example III-2 in a determined amount to measure a reactivity in the conversion into a polyurethane. Results in the measurements are shown in Table IV-1 and Table IV-2.

<Comparative Example IV-1>

The same procedures were repeated as in the Example IV-1 except that dibutyltin dilaurate was not added to MDMCHDI obtained by a DMC method in Production Example III-2 to measure a reactivity in the conversion into a polyurethane. Results in the measurements are shown in Table IV-2.

<Comparative Example IV-2>

The same procedures were repeated as in the Example IV-1 except that MDMCHDI prepared by a phosgene method in the Example I-2 was employed, and dibutyltin dilaurate was not employed to measure a reactivity in the conversion into a polyurethane. Results of the measurements are shown in Table IV-2.

<Example IV-7>

A reaction apparatus equipped with a condenser, an agitator, thermometer, and a tube for supplying nitrogen gas was charged with 104.1 g of MDMCHDI obtained in the above-described Production Example III-2, $3.5 \times 10^{-5}$ equivalent of acetic acid based on isocyanate group, and 1,000 g of a polytetramethyleneglycol having a number average molecular weight of 2,000 (PTG 2000 manufactured by Nihon Polyurethane Kogyo Co., Ltd.), followed by being immediately heated and allowed to react for the conversion into a polyurethane at 120°C.

Residual amount of MDMCHDI in a reactant was monitored with a lapse of time by a gas chromatographic analysis method to measure the conversion into a polyurethane. As a result, even though the reaction was continued for 3 hours after attained to a reaction temperature of 120°C, a large decrease was not observed in the concentration of the residual MDMCHDI and the concentration of isocyanate group.

Results in the measurements are shown in Table IV-2.

<Example IV-8>

The same conditions in the conversion into a polyurethane were followed as in the Example IV-7, except that a reaction temperature was changed to 70°C.

Residual amount of MDMCHDI in a reactant was monitored with a lapse of time by a gas chromatographic analysis method to measure the conversion into a polyurethane. As a result, even though the reaction was continued for 9 hours after attained to a reaction temperature of 70°C, a large decrease was not observed in the concentration of the residual MDMCHDI and the concentration of isocyanate group.

Results of the measurements are shown in Table IV-2.

Table IV-1

| | EXAMPLE IV-1 | EXAMPLE IV-2 | EXAMPLE IV-3 | EXAMPLE IV-4 | EXAMPLE IV-5 |
|---|---|---|---|---|---|
| A | D-MDMCHDI | D-MDMCHDI | D-MDMCHDI | D-MDMCHDI | D-MDMCHDI |
| B | DBTDL | MA | TC | BE | AC |
| C | 1.12 | 6.71 | 3.65 | 3.16 | 2.50 |
| D | 1.0 | 1.0 | 1.0 | 1.0 | 1 0 |
| E | 60 | 60 | 60 | 60 | 60 |
| F | 2.13 | 2.07 | 2.16 | 2.2 | 1.51 |

A: Diisocyanate compound
B:Kind of Lewis acids
C:Equivalent of Lewis acids added (equivalent/$10^{-4}$ equivalent of isocyanate group)
D:Mixing ratio of NCO/OH in the urethanation reaction
E:Reaction temperature (°C)
F:Reaction proportion of MDMCHDI after 3 hours (based on Comparative Example 1)

Table IV-2

| | EXAMPLE IV-6 | EXAMPLE IV-7 | EXAMPLE IV-8 | COMPARATIVE EXAMPLE IV-1 | COMPARATIVE EXAMPLE IV-2 |
|---|---|---|---|---|---|
| A | D-MDMCHDI | D-MDMCHDI | D-MDMCHDI | D-MDMCHDI | P-DMCHDI |
| B | TBT | DBTDL | DBTDL | -- | -- |
| C | 0.92 | 0.01 | 11.5 | -- | -- |
| D | 1.0 | 1.0 | 1.0 | 1.0 | 1.0 |
| E | 60 | 120 | 70 | 60 | 60 |
| F | 2.09 | 1.6 | >2.2 | 1 | 1.93 |

A:Diisocyanate compound
B:Kind of Lewis acids
C:Equivalent of Lewis acids added (equivalent/$10^{-4}$ equivalent of isocyanate group)
D:Mixing ratio of NCO/OH in the conversion into a polyurethane
E:Reaction temperature (°C)
F:Reaction proportion of MDMCHDI after 3 hours (based on Comparative Example 1)

In the Table IV-1 and Table IV-2, abbreviations are as follows.

D-MDMCHDI: 3-isocyanatemethyl-5,5-dimethylcyclohexyl isocyanate prepared by a dimethyl carbonate method
P-MDMCHDI: 3-isocyanatemethyl-5,5-dimethylcyclohexyl isocyanate prepared by a phosgene method
DBTDL: dibutyltin dilaurate
TBT: tetrabutyl titanate
MA: manganese acetate
SC: stannous chloride
BE: borontrifluoride diethyletherate ($BF_3Et_2O$)
ATC: aluminum trichloride

<Example IV-9>

The same conditions in the conversion into a polyurethane were followed as in the Example IV-7, except that a polycaprolactone diol having a number average molecular weight of 2,000 (Placcel 220 manufactured by Daicel Chemical Industries, Ltd.) was employed in place of the polytetramethyleneglycol.

Residual amount of MDMCHDI in a reactant was monitored with a lapse of time by a gas chromatographic

analysis method to measure the conversion into a polyurethane. As a result that the reaction was continued, there was obtained a urethane polymer having the concentration of the residual MDMCHDI of 4.0% by weight and the concentration of isocyanate group of 3.6% by weight at a period of 2 hours after attained to a reaction temperature of 120°C.

After that, although the reaction was continued, a large decrease was not observed in the concentration of the residual MDMCHDI and the concentration of isocyanate group.

<Example IV-10>

A reaction apparatus equipped with a condenser, an agitator, a dropping funnel, thermometer, and a tube for supplying nitrogen gas was charged with 2,000.0 g of MDMCHDI obtained in Production Example III-2, $3.5x10^{-5}$ equivalent of manganese acetate based on isocyanate group, followed by heating to 50°C. 190.8 g of trimethylolpropane (manufactured by Mitsubishi Gas Kagaku Kogyo, Ltd.) thermally melted at 70°C was added dropwise over 3 hours, and reaction temperature elevated to 70°C.

After the completion of dropwise addition, the concentration of isocyanate group in a reactant was monitored with a lapse of time by a titration analysis method to measure the conversion into a polyurethane. At a period of 3 hours after the completion of dropwise addition, there was obtained a mixture of a urethane polymer/monomer having the concentration of isocyanate group of 29.2% by weight. After that, although the reaction was continued, a large decrease was not observed in the concentration of isocyanate group.

<Comparative Example IV-3>

A reaction apparatus equipped with a condenser, an agitator, thermometer, and a tube for supplying nitrogen gas was charged with 104.1 g of MDMCHDI obtained in Production Example III-2 and 1,000 g of a polytetramethyleneglycol having a number average molecular weight of 2,000 (PTG 2000 manufactured by Nihon Polyurethane Kogyo Co., Ltd.), followed by being immediately heated and allowed to react for the conversion into a polyurethane at 120°C.

As a result, even though the reaction was continued for 6 hours after attained to a reaction temperature of 120°C, a large decrease was not observed in the concentration of the residual MDMCHDI and the concentration of isocyanate group.

<Comparative Example IV-4>

The same conditions in the conversion into a polyurethane were followed as in the Comparative Example IV-3, except that a reaction temperature was changed to 70°C.

Residual amount of MDMCHDI in a reactant was monitored with a lapse of time by a gas chromatographic analysis method to measure the conversion into a polyurethane. As a result, there was obtained a urethane polymer having the concentration of the residual MDMCHDI of 7.7% by weight and the concentration of isocyanate group of 4.1% by weight at a period of 12 hours after attained to a reaction temperature of 70°C, which was not a desired urethane prepolymer.

<Comparative Example IV-5>

The same conditions in the conversion into a polyurethane were followed as in the Comparative Example IV-4, except that a polycaprolactone diol having a number average molecular weight of 2,000 (Placcel 220 manufactured by Daicel Chemical Industries, Ltd.) was employed in place of the polytetramethyleneglycol.

Residual amount of MDMCHDI in a reactant was monitored with a lapse of time by a gas chromatographic analysis method to measure the conversion into a polyurethane. As a result, there was obtained a urethane prepolymer having the concentration of the residual MDMCHDI of 3.8% by weight and the concentration of isocyanate group of 3.4% by weight at a period of 4 hours after attained to a reaction temperature of 120°C. After that, although the reaction was continued, a large decrease was not observed in the concentration of the residual MDMCHDI and the concentration of isocyanate group.

<Comparative Example IV-6>

A reaction apparatus equipped with a condenser, an agitator, a dropping funnel, a thermometer, and a tube for supplying nitrogen gas was charged with 2,000 g of MDMCHDI obtained in Production Example III-2, followed by being heated to 50°C. 190.8 g of trimethylolpropane (manufactured by Mitsubishi Gas Kagaku Kogyo, Ltd.) thermally melted at 70°C was added dropwise over 3 hours, and reaction temperature elevated to 70°C.

After the completion of dropwise addition, the concentration of isocyanate group in a reactant was monitored

with a lapse of time by a titration analysis method to measure the urethanation reaction. At a period of 9 hours after the completion of dropwise addition, although there was obtained a mixture of a urethane prepolymer/monomer having the concentration of isocyanate group of 31.4% by weight, viscosity is very high in the mixture of a urethane polymer/monomer which has a large molecular weight distribution compared to a desired mixture of a urethane prepolymer/monomer.

<Comparative Example IV-7>

The same conditions in the conversion into a polyurethane were followed as in the Comparative Example IV-4, except that MDMCHDI by a phosgene method obtained in Example I-2 was employed. At a period of 4 hours after attained to a reaction temperature of 120°C, there was obtained a urethane polymer having the concentration of the residual MDMCHDI of 4.0% by weight and the concentration of isocyanate group of 3.5% by weight.
After that, although the reaction was continued, a large decrease was not observed in the concentration of the residual MDMCHDI and the concentration of isocyanate group.
It is to be noted that in the Table IV-1 and Table IV-2, reaction proportion of MDMCHDI after 3 hours is the reaction proportion based on 1 of that of MDMCHDI in the Comparative Example IV-1.
By the addition of the Lewis acids as in the present invention, a reactivity in the polyisocyanate compounds prepared from a diamine and a dialkylcarbonate substantially prepared without using phosgene through all steps is accelerated in an identical or more extent of polyisocyanate compounds prepared through conventional preparation methods by phosgene. And, it is distinct that there can be obtained an excellent weatherability, corrosion resistance, and heat resistance.

[Examples for the fifth aspect of the present invention]

<Example V-1/Preparation of a polyurethane>

A reaction apparatus equipped with a condenser, an agitator, thermometer, and a tube for supplying nitrogen gas was charged with 41.4 g of MDMCHDI obtained by a carbamate method in Example I-1 and 200 g of a polycaprolactone diol PCL-220, and reaction temperature was set up at 80°C while agitating under a nitrogen atmosphere.
Reaction for the conversion into a polyurethane was continued for 3 hours to obtain a urethane prepolymer having the NCO content of 3.3% by weight.
After temperature in the reaction apparatus was cooled to 40°C, a homogeneous solution was prepared by mixing the urethane prepolymer with 199 g of xylene and 161 g of methylisobutylketone (MIBK).
A reaction for extending chains by a diamine was carried out by adding 15.4 g of MDMCHDA obtained in Synthesis Example I-2, 0.8 g of diisobutylamine, 38.0 g of MIBK, and 199 g of isopropanol to a solution of the urethane prepolymer obtained.
Reaction temperature was set up at 50°C, and the reaction was continued for 3 hours to obtain a solution of the polyurethane prepolymer. The solution of the polyurethane prepolymer obtained can be quickly cured, and it is useful as an adhesive having excellent weatherability, heat resistance, and corrosion resistance.

<Example V-2/Preparation of a polyurethane>

The same procedures were followed using the same apparatus as in the Example V-1, except that 41.6 g of MDMCHDI by a phosgene method obtained in Example I-2 was employed as a diisocyanate, and 200 g of the polytetramethyleneglycol PTG2000 was employed in place of the polycaprolactone diol PCL-220 to prepare a solution of a polyurethane resin.
The solution of a polyurethane resin obtained can be quickly cured, and it is useful as an adhesive having excellent weatherability, heat resistance, and corrosion resistance.

[Examples for the sixth aspect of the present invention]

<Example VI-1/Preparation of a polyurethane resin solution>

A reaction apparatus equipped with a condenser, an agitator, thermometer, and a tube for supplying nitrogen gas was charged with 41.4 g of MDMCHDI obtained in Production Example III-2, 200 g of the polycaprolactone diol PCL-220, and 46.2 mg ($1.12x10^{-4}$ equivalent based on isocyanate group) of dibutyltin dilaurate, and reaction temperature was set up at 80°C while agitating under a nitrogen atmosphere. Reaction for the conversion into a polyurethane was continued for 3 hours to obtain a urethane prepolymer having the NCO content of 3.3% by weight.

After temperature in the reaction apparatus was cooled to 40°C, a homogeneous solution was prepared by mixing the urethane prepolymer with 199 g of xylene and 161 g of methylisobutylketone (MIBK).

A reaction for extending chains by a diamine was carried out by adding 15.4 g of MDMCHDA obtained in Production Example I-2, 0.7 g of diisobutylamine, 38.0 g of MIBK, and 199 g of isopropanol to a urethane prepolymer solution obtained.

Reaction temperature was set up at 50°C, and the reaction was continued for 3 hours to obtain a desired polyurethane resin solution.

The polyurethane resin solution obtained showed solid content of 30.8% by weight, a viscosity of 4,300 cP/25°C, and residual NCO of less than 0.05 % by weight.

<Example VI-2/Preparation of a polyurethane resin composition>

The same procedures were followed using the same apparatus as in the Example VI-1, except that 200 g of the polytetramethylene glycol PTG2000 was employed in place of the polycaprolactonediol PCL-220 to prepare a polyurethane resin solution.

The polyurethane resin solution obtained showed solid content of 31.5% by weight, a viscosity of 3,900 cP/25°C, and residual NCO of less than 0.04 % by weight.

<Comparative Example VI-1/Preparation of a resin composition>

The same procedures were followed using the same apparatus as in the Example VI-1, except that the MDMCHDI obtained in the Example I-2 was employed in place of the MDMCHDI obtained in the Production Example III-2 to prepare a polyurethane resin solution.

The polyurethane resin solution obtained showed solid content of 30.6% by weight, a viscosity of 4,060 cP/25°C, and residual NCO of less than 0.01 % by weight.

<Comparative Example VI-2/Preparation of a resin composition>

The same procedures were followed using the same apparatus as in the Example VI-2, except that the MDMCHDI by a phosgene method obtained in the Example I-2 was employed in place of the MDMCHDI obtained in the Production Example III-2 to prepare a polyurethane resin solution.

The polyurethane resin solution obtained showed solid content of 31.2% by weight, a viscosity of 2,160 cP/25°C, and residual NCO of less than 0.01 % by weight.

<Example VI-3/Preparation of a urethane prepolymer>

A reaction apparatus equipped with a thermometer, an agitator, a tube for supplying nitrogen gas, and a condenser was charged with 1968 g of PPTG2000 which is a tetrahydrofran-propylene oxide copolymer, 416 g of MDMCHDI obtained in Production Example III-2, 0.1 g of dibutyltin dilaurate, and 0.5 g of paramethyl benzoic acid, followed by allowing to react at 60-70°C for 5 hours to obtain a urethane prepolymer.

Temperature was lowered to 50°C, and 1.3 g of t-butylhydroquinone, 255 g of 2-hydroxyethylacrylate, and 0.3 g of dibutyltin dilaurate were added and allowed to react for 5 hours to obtain a urethane acrylate resin.

20 g of trimethylolpropane triacrylate, 5 g of benzyldimethyl ketal, and 5 g of benzophenone were mixed with 70 g of the urethane acrylate resin to obtain a resin composition for an ultraviolet ray-curable type adhesive.

<Comparative Example VI-3/Preparation of a urethane resin composition>

The same procedures were followed using the same apparatus as in the Example VI-3, except that the MDMCHDI obtained by a phosgene method in Example I-2 was employed in place of the MDMCHDI obtained in Production Example III-2 to obtain a resin composition for an ultraviolet ray-curable type adhesive.

<Example VI-4/Preparation of a resin solution of a urethane prepolymer>

A reaction apparatus equipped with a thermometer, an agitator, a tube for supplying nitrogen gas, and a condenser was charged with 60 g of xylene and 0.2 g of benzoyl peroxide, followed by elevating temperature to 120°C.

Subsequently, there was added dropwise a mixed solution composed of 15.6 g of styrene, 30.0 g of methylmethacrylate, 19.2 g of n-butylacrylate, 37.3 g of gamma-methacryloxypropyltrimethoxy silane, and 0.1 g of azobisisobutyronitrile at 120-130°C over 2 hours.

Reaction was further continued for 3 hours at the same temperature, followed by cooling the reaction temperature to 70°C, and by adding 52 g of Sannix PP-400 which is a polypropyleneglycol, 27.1 g of the MDMCHDI obtained in Production Example III-2, and 0.02 g of dibutyltin dilaurate to allow to react for 4 hours at the same temperature, and then adding 2.0 g of endoethylenepiperadine, and 2.0 g of dibutyltin diacetate to obtain a resin solution of a urethane prepolymer having isocyanate groups.

<Comparative Example VI-4/Preparation of a resin solution of a urethane prepolymer>

The same procedures were followed using the same apparatus as in the Example VI-4, except that the MDMCHDI obtained by a phosgene method in Example I-2 was employed in place of the MDMCHDI obtained in Production Example III-2 to obtain a resin solution of a urethane prepolymer.

<Example VI-5/Preparation of a laminated film>

PTG1000 which is a polytetramethyleneglycol, 1,4-butanediol, and Placcel 305 which is a polycaprolactone triol were mixed in a mixing ratio of 35:55:10 by weight, followed by adding 0.05% by weight of dibutyltin dilaurate to prepare a polyol mixture.

Separately, a urea derivative of the NCO content of 31.5% containing an isocyanate group was prepared using the MDMCHDI obtained in Production Example III-2 by a method described in JP-B-83040536 Official Gazette.

The above-described polyol mixture and urea derivative were mixed in a mixing ratio of NCO/OH=1.0 (by mol), followed by defoaming in vacuo and casting on a polycarbonate resin film at approximately 40°C. The film was dried and cured at approximately 120°C for 25 minutes to obtain a film having the thickness of approximately 0.5 mm.

<Comparative Example VI-5>

The same procedures were followed using the same apparatus as in the Example VI-5, except that-the MDMCHDI obtained by a phosgene method in Example 1-2 was employed in place of the MDMCHDI obtained in Production Example III-2 to obtain a film.

<Application Example VI-1>

A mixture composed of 50 parts by weight of titanium oxide (a trade name, Typaque CR-90 manufactured by Ishihara Sangyo Co. Ltd.), 70 parts by weight of an MIBK/toluene/IPA mixture (weight ratio of 30:30:40), 0.75 part by weight of Disparlon 1860 (a leveling agent manufactured by Kusumoto Kasei Co. Ltd.), 0.75 part by weight of Disparlon 1984-50 (a wetting dispersant, and 0.75 part by weight of Disparlon OX-77 (an antifoaming agent manufactured by Kusumoto Kasei Co. Ltd.) was mixed with 100 parts by weight of the respective resin solution of a polyurethane obtained in Example VI-1, Example VI-2, Comparative Example VI-1, and Comparative Example VI-2 to prepare a composition. The composition was dispersed together with 150 parts by weight of glass media having a particle diameter of 3 mm using a vibrating dispersion mill for approximately 1 hour to prepare a white-colored printing ink. The white-colored printing ink obtained was coated on an OPP film and a PET film using a No. 40 bar coater and dried at room temperature and a humidity of 60-70% for 1 week.

Resulting printed products were evaluated for adhesion, heat resistance, and humidity resistance, and results are shown in Table VI-1. The adhesive comprising a polyurethane resin composition according to the present invention is more improved in physical properties such as weatherability and heat resistance.

Table VI-1

| (Results in Application Example VI-1) | | | | |
|---|---|---|---|---|
| Polyurethane | EXAMPLE VI-1 | EXAMPLE VI-2 | COMPARATIVE EXAMPLE VI-1 | COMPARATIVE EXAMPLE VI-2 |
| Resin solution | 100 | 100 | 100 | 100 |
| A | 50 | 50 | 50 | 50 |
| B | 70 | 70 | 70 | 70 |
| C | 0.75 | 0.75 | 0.75 | 0.75 |
| D | 0.75 | 0.75 | 0.75 | 0.75 |

Table VI-1 (continued)

| (Results in Application Example VI-1) | | | | |
|---|---|---|---|---|
| Polyurethane | EXAMPLE VI-1 | EXAMPLE VI-2 | COMPARATIVE EXAMPLE VI-1 | COMPARATIVE EXAMPLE VI-2 |
| E | 0.75 | 0.75 | 0.75 | 0.75 |
| Base material | OPP PET | OPP PET | OPP PET | OPP PET |
| Adhesion test | Δ o | Δ o | Δ o | Δ o |
| Hot water test | Δ o | Δ Δ | Δ Δ | x Δ |
| Heat resistance test | Δ Δ | x Δ | x Δ | x x |
| Accelerated weatherability test | Δ Δ | x Δ | x Δ | x x |

In the Table VI-1, abbreviations, testing methods, and standards for evaluation are as follows.

A: Typaque CR-90
B: Thinner
C: Disparlon 1860
D: Disparlon 1984-50
E: Disparlon OX-77
OPP: oriented polypropylene film
PET: polyester film
Adhesion test: 90°-peeling test by a Cellophane tape
o: no peeled, Δ: partially peeled, x: completely peeled
Hot water resistance test: adhesion test after immersion for 2 hours in hot water of 80°C
Heat resistance test: adhesion test after 60 hours at 120°C
Accelerated weatherability test: adhesion test after 200 hours exposure (dew condensation/exposure=2/10 hours cycle) with a dew panel weatherometer (manufactured by Suga Shikenki Co. Ltd.)

<Application Example VI-2>

The respective ultraviolet ray-curable type adhesive compositions obtained in the Example VI-3 and the Comparative Example VI-3 were coated on a polyvinylchloride sheet with No. 20-bar coater, and then an OPP sheet on which a chlorinated polypropylene resin is coated was laminated to obtain a laminated sheet.

The laminated sheet was cured using a converging-type high pressure mercury lamp (manufactured by Iwasaki Denki Co. Ltd.) equipped with a reflection plate having an irradiation power of 1 kW and power density of 40 W/cm in a conveyer speed of 10 m/minute to obtain a cured sheet.

Table VI-2 shows evaluation results in relation to adhesion, weatherability, and heat resistance in the cured sheets obtained above. The adhesive comprising a polyurethane resin composition of the present invention has more improved physical properties such as adhesion, weatherability, and heat resistance.

Table VI-2

| (Results in Application Example VI-2) | | |
|---|---|---|
| Ultraviolet ray-curable type compositions | EXAMPLE VI-3 | COMPARATIVE EXAMPLE VI-3 |
| Adhesive strength (kgf/15 mm) | 78 | 76 |
| Hot water resistance test (%) | 88 | 65 |
| Heat resistance test (%) | 82 | 76 |
| Accelerated weatherability test | 75 | 73 |

In the Table VI-2, test conditions are as follows.

Hot water resistance test: adhesion test after immersion for 48 hours in hot water of 80°C

Heat resistance test: adhesion test after 60 hours at 120°C
Accelerated weatherability test: retention ratio of an adhesive strength after 200 hours exposure (dew condensation/exposure=2/10 hours cycle) with a dew panel weatherometer (manufactured by Suga Shikenki Co. Ltd.)

<Application Example VI-3>

The respective urethane prepolymer solutions obtained in the Example VI-4 and the Comparative Example VI-4 were coated on a clean aluminum plate with No. 20-bar coater, and then a polyester non-woven cloth sheet was laminated to obtain a laminated sheet.
The laminated sheet was cured under the conditions of 20°C and humidity of 70% to obtain a moisture curable-type laminated sheet.
Table VI-3 shows evaluation results in relation to adhesion, weatherability, and heat resistance of the cured sheet obtained above. The adhesive comprising a polyurethane resin composition of the present invention has more improved physical properties such as adhesion, weatherability, and heat resistance.

Table VI-3

| (Results in Application Example VI-3) | | |
|---|---|---|
| Polyurethane prepolymer | EXAMPLE VI-4 | COMPARATIVE EXAMPLE VI-4 |
| Adhesive strength (kgf/15 mm) | 68 | 68 |
| Hot water resistance test (%) | 45 | 42 |
| Heat resistance test (%) | 62 | 67 |
| Accelerated weatherability test | 85 | 78 |

In the Table VI-3, test conditions are as follows.

Hot water resistance test: adhesion test after immersion for 48 hours in hot water of 80°C
Heat resistance test: adhesion test after 60 hours at 120°C
Accelerated weatherability test: retention ratio of an adhesive strength after 200 hours exposure (dew condensation/exposure=2/10 hours cycle) with a dew panel weatherometer (manufactured by Suga Shikenki Co. Ltd.)

<Application Example VI-4>

The laminated films obtained in the Example VI-5 and the Comparative Example VI-5 were come in contact with a clean aluminum plate, followed by laminating at 80°C for 30 minutes while compressing at 5 kg/cm to obtain an aluminum laminated sheet.
Table VI-4 shows evaluation results in relation to adhesion, weatherability, and heat resistance of the laminated sheet obtained above. The adhesive comprising a polyurethane resin composition of the present invention has more improved physical properties such as adhesion, weatherability, and heat resistance.

Table VI-4

| (Results in Application Example VI-4) | | |
|---|---|---|
| Lamination sheet | EXAMPLE VI-5 | COMPARATIVE EXAMPLE VI-5 |
| Shear strength (kgf/15 mm) | 150 | 145 |
| Hot water resistance test (%) | 86 | 84 |
| 86Heat resistance test (%) | 78 | 76 |
| Accelerated weatherability test | 78 | 67 |

According to the first aspect of the present invention, the novel cycloaliphatic polyisocyanate has a high reactivity because of methyl group not substituted at a substitution position of isocyanatemethyl group.
According to the second aspect of the present invention, corrosion resistance is improved in a urethane coating and other polyurethane products using the cycloaliphatic polyisocyanate having the chlorine content of only less than 10 ppm.

Further, a catalyst for the conversion into a polyurethane is not hindered by chlorine ion. Accordingly, the use amount of the catalyst can be decreased. As a result, weatherability, moisture resistance, and heat resistance are improved in polyurethanes owing to a low content of amines and metal salts in the urethane coating and other polyurethane products.

According to the third aspect of the present invention, a reactivity in the conversion into a polyurethane can be controlled in a polyisocyanate compound having a low reactivity. That is, a reactivity in the conversion into a polyurethane can be controlled by adding protonic acids into a polyisocyanate prepared from a dialkylcarbonate and a diamine, whereas it is difficult to control a reactivity in a polyisocyanate compound prepared by a conventional phosgene method using diamines. Further, there can be obtained a polyurethane having more excellent weatherability, corrosion resistance, and heat resistance compared to a conventional polyurethane owing to not containing impurities derived from phosgene.

According to the fourth aspect of the present invention, a technology for elevating a reactivity of the cycloaliphatic polyisocyanate was completed, and there can be obtained a polyurethane having more improved physical properties such as weatherability, corrosion resistance, and heat resistance, and which can be preferably employed for coatings, adhesives, and molded articles.

According to the fifth aspect of the present invention, a polyurethane obtained using the above-described cycloaliphatic polyisocyanate are useful as coatings and adhesives.

According to the sixth aspect of the present invention, there can be obtained an adhesive having more improved physical properties such as weatherability, corrosion resistance, and heat resistance.

While the invention has been described in detail and with reference to specific embodiments thereof, it will be apparent to one skilled in the art that various changes and modifications can be made therein without departing from the spirit and scope thereof.

## Claims

1. A cycloaliphatic polyisocyanate represented by general formula (1) described below,

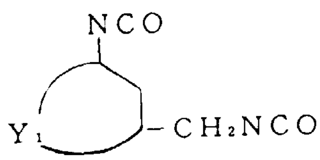

(1)

in which Y1 is a divalent saturated aliphatic hydrocarbon residual group.

2. A cycloaliphatic polyisocyanate as claimed in claim 1, wherein said Y1 in the formula (1) is an alkylene group having a carbon number of 1-10.

3. A cycloaliphatic polyisocyanate as claimed in claim 1, wherein said cycloaliphatic polyisocyanates represented by the general formula (1) is 3-isocyanatemethyl-5,5-dimethylcyclohexylisocyanate.

4. A cycloaliphatic polyisocyanate according to one of claims 1 to 3, characterized in that the chlorine content is not more than 10 ppm.

5. A process for the preparation of a cycloaliphatic polyisocyanate represented by general formula (1) as claimed in claim 1, characterized by further subjecting 3-aminomethylcycloalylamines represented by general formula (4) to a reaction for conversion into an isocyanate after a reductive amination of 3-formylcycloalkanones represented by general formula (2) or 3-formylcycloalkenones represented by general formula (3) ;

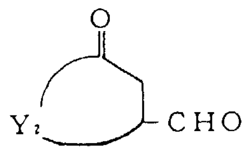

(2)

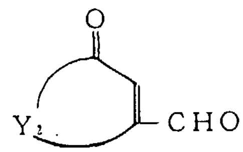

(3)

in which Y2 is a divalent saturated or unsaturated aliphatic hydrocarbon residual group

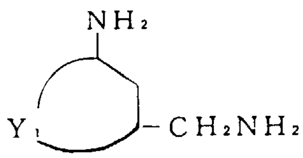

(4)

in which Y1 is a divalent saturated aliphatic hydrocarbon residual group.

**6.** A process according to claim 5 for the preparation of cycloaliphatic polyisocyanates as claimed in one of claims 1 to 4, wherein said reductive amination is carried out by introducing hydrogen into a reaction system containing 3-formylcycloalkanones or 3-formylcycloalkenones, ammonia, and a solvent under the presence of a catalyst.

**7.** A process for the preparation of cycloaliphatic polyisocyanates according to claim 5 or 6, wherein there are employed 3-formylcycloalkanones represented by general formula (2) or 3-formylcycloalkenones represented by general formula (3) produced by oxidation of 3-methylcycloakanones represented by general formula (5) or 3-methylcycloalkenones represented by general formula (6):

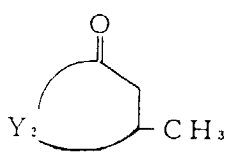

(5)

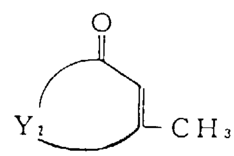

,

(6)

in which Y2 is a divalent saturated or unsaturated aliphatic hydrocarbon residual group.

**8.** A process for the preparation of a cycloaliphatic polyisocyanate as claimed in claim 5, characterized in that said reaction for conversion into an isocyanate is carried out by thermal decomposition of a dialkylcarbamate represented by general formula (7) obtained by a reaction of 3-aminomethylcycloalkyl amines with a dialkylcarbonate prepared without using phosgene through a reaction step;

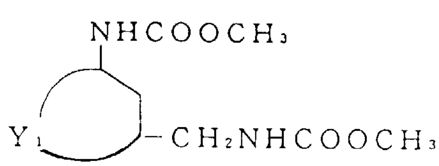

(7)

in which, Y1 is a divalent saturated aliphatic hydrocarbon residual group.

**9.** A process for the preparation of the cycloaliphatic polyisocyanate according to claim 8, characterized in that said alkylcarbonate is dimethylcarbonate.

**10.** A process for the preparation of the cycloaliphatic polyisocyanate according to claim 9, characterized in that said alkylcarbonate is dimethylcarbonate which is prepared from methanol, oxygen, and carbon monoxide.

**11.** A process for the preparation of the cycloaliphatic polyisocyanate as claimed in claim 9, characterized in that said alkylcarbonate is dimethylcarbonate which is prepared by a further reaction of propylene carbonate with methanol, and propylene carbonate is prepared from propylene and carbondioxide.

**12.** A cycloaliphatic polyisocyanate composition primarily containing a cycloaliphatic polyisocyanate as claimed in any one of claims 1-4, and containing a protonic acid and/or a Lewis acid.

**13.** A cycloaliphatic polyisocyanate composition according to claim 12 primarily containing a cycloaliphatic polyisocyanate as claimed in any one of claims 1-4, and containing a Lewis acid.

**14.** A cycloaliphatic polyisocyanate composition primarily containing a cycloaliphatic polysicyanate as claimed in claim 4 and containing a Lewis acid.

**15.** A cycloaliphatic polyisocyanate composition as claimed in one of claims 12 to 14, wherein the amount of the protonic acids and/or Lewis acids to be added is $1x10^{-8}$-$1x10^{-1}$ based on 1 equivalent of isocyanate group in said cycloaliphatic polyisocyanate.

**16.** A polyurethane obtained by a reaction of the cycloaliphatic polyisocyante as claimed in any one of claims 1-4 with polyols and/or polyamines.

**17.** A polyurethane obtained by a reaction of the cycloaliphatic polyisocyanate as claimed in any one of claims 12-14 with polyols and/or polyamines.

**18.** An adhesive composition comprising a polyurethane as claimed in any one of claims 16-17.